(19) Europäisches Patentamt
European Patent Office
Office européen des brevets

(11) EP 4 332 084 A1

(12) **EUROPEAN PATENT APPLICATION**
published in accordance with Art. 153(4) EPC

(43) Date of publication:
**06.03.2024 Bulletin 2024/10**

(21) Application number: **22795689.3**

(22) Date of filing: **22.04.2022**

(51) International Patent Classification (IPC):
***C07C 68/08*** *(2006.01)* ***C07C 69/96*** *(2006.01)*

(52) Cooperative Patent Classification (CPC):
(C-Sets available)
**C07C 68/08;** Y02E 60/10 (Cont.)

(86) International application number:
**PCT/JP2022/018599**

(87) International publication number:
**WO 2022/230776 (03.11.2022 Gazette 2022/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.04.2021 JP 2021075935**

(71) Applicant: **Asahi Kasei Kabushiki Kaisha**
**Tokyo 1000006 (JP)**

(72) Inventors:
- **ENOMOTO, Miyako**
  **Tokyo 100-0006 (JP)**
- **OCHI, Hiroyuki**
  **Tokyo 100-0006 (JP)**

(74) Representative: **Strehl Schübel-Hopf & Partner**
**Maximilianstrasse 54**
**80538 München (DE)**

(54) **METHOD FOR MANUFACTURING DIALKYL CARBONATE AND DEVICE FOR MANUFACTURING DIALKYL CARBONATE**

(57) A production method of a dialkyl carbonate, comprising two separation and purification steps (I) and (II), wherein, in the step (I), the concentration of the dialkyl carbonate in the mixture ($A_T$) is from 25.00 to 95.00% by mass, and the temperature of column bottom is 115°C or more, and in the step (II), the concentration of the dialkyl carbonate in the column bottom component ($B_B$) is from 99.00 to 99.95% by mass, and the purity of the dialkyl carbonate in the side-cut component ($B_s$) is 99.99% by mass or more.

[Figure 1]

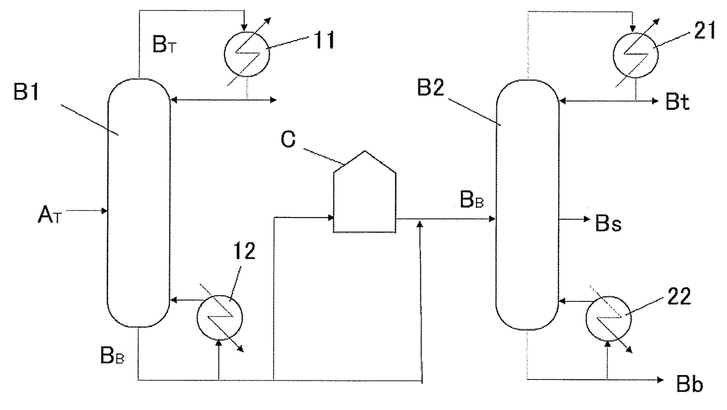


EP 4 332 084 A1

(52) Cooperative Patent Classification (CPC): (Cont.)

C-Sets
**C07C 68/08, C07C 69/96**

## Description

### Technical Field

The present invention relates to a production method of a dialkyl carbonate and a production apparatus for a dialkyl carbonate.

### Background Art

Dialkyl carbonates have been used as major components for lithium ion battery electrolyte solutions in recent years. As industries of electronic products and clean energy automobiles develop, the importance of lithium ion battery electrolyte solutions is increasingly growing. Accordingly, market demands for high-purity dialkyl carbonates for lithium ion battery electrolyte solutions have greatly increased. Industry-grade dialkyl carbonates contain impurities such as water, methanol, ethanol, ethyl methyl carbonate, and heavy components. Alcohols and water affect the service life of lithium ion batteries, and heavy components affect discoloration of electrolyte solutions. Thus, dialkyl carbonates for lithium ion battery electrolyte solutions are required to have a further reduced content of these impurities and to have an extremely high purity (a purity of 99.99% by mass or more).

Some methods for producing such a high-purity dialkyl carbonate for lithium ion battery electrolyte solutions have been proposed. For example, methods have been proposed in which two distillation columns are used, industry-grade (a purity of 99.95% by mass) dimethyl carbonate is fed to the first column, and high-purity (a purity of 99.99% by mass or more) dimethyl carbonate is obtained from the side-cut of the second column (e.g., see Patent Literatures 1 and 2).

### Citation List

#### Patent Literature

Patent Literature 1: Chinese Patent Application Publication No. 107311864
Patent Literature 2: Chinese Patent Application Publication No. 105384639

### Summary

#### Technical Problem

The methods described in Patent Literatures 1 and 2, however, leave room for improvement, because the reflux ratio is as large as 20 to 40 both in the first and second distillation columns and a large amount of heat consumption is necessary for producing a high-purity dialkyl carbonate for lithium ion battery electrolyte solutions.

For example, distilling a crude dialkyl carbonate obtained in a production step with the first distillation column provides a dialkyl carbonate having a purity of 99.00 to 99.95% by mass, and the dialkyl carbonate having the purity is utilized as an industry-grade dialkyl carbonate for use in various industrial applications.

Subsequently, the dialkyl carbonate having a purity of 99.00 to 99.95% by mass is introduced to the second distillation column to provide a high-purity dialkyl carbonate having a purity of 99.99% by mass or more. A high-purity dialkyl carbonate has a purity which enables the dialkyl carbonate to be used for lithium ion battery electrolyte solutions. The second distillation column to provide a high-purity dialkyl carbonate requires a high amount of heat consumption in order to elevate the purity to a desired level.

It is thus an object of the present invention to provide a production method of a dialkyl carbonate, which method reduces the amount of heat consumption in a separation and purification step of providing a high-purity dialkyl carbonate having a purity of 99.99% by mass or more from a dialkyl carbonate having a purity of 99.00 to 99.95% by mass, and a production apparatus for a dialkyl carbonate.

#### Solution to Problem

As a result of intensive studies to solve the problems, the present inventors have found that by not raising but rather lowering the purity of a dialkyl carbonate to be fed to a first distillation column for purifying a dialkyl carbonate and additionally by setting the temperature of column bottom of the first distillation column to 115°C or more, separation and purification of a high-purity dialkyl carbonate having a purity of 99.99% by mass or more from a dialkyl carbonate having a purity of 99.00 to 99.95% by mass in a second distillation column can be done with a reduced amount of heat consumption, that is, the dialkyl carbonate can be produced at a smaller reflux ratio having completed the present invention.

That is, the present invention relates to embodiments below.

<1> A production method of a dialkyl carbonate, comprising:

(I) a first separation and purification step (I) of continuously feeding a low-purity dialkyl carbonate mixture ($A_T$) containing a dialkyl carbonate and an aliphatic monohydric alcohol to a continuous multi-stage distillation column B1, continuously withdrawing a column top component ($B_T$) containing the aliphatic monohydric alcohol as the main component from the upper portion of the column, and continuously withdrawing a column bottom component ($B_B$) containing the dialkyl carbonate as the main component from the lower portion of the column, and
(II) a second separation and purification step (II) of continuously feeding the column bottom component ($B_B$) containing the dialkyl carbonate as the main component continuously withdrawn from the column bottom portion of the continuous multi-stage distillation column B1 to a continuous multi-stage distillation column B2 having a side withdrawal port, and continuously withdrawing a side-cut component ($B_S$) containing the dialkyl carbonate as the main component from the side withdrawal port, wherein

in the step (I), a concentration of the dialkyl carbonate in the low-purity dialkyl carbonate mixture ($A_T$) to be fed to the continuous multi-stage distillation column B1 is from 25.00 to 95.00% by mass, and a temperature of column bottom of the continuous multi-stage distillation column B1 is 115°C or more,
in the step (II), a concentration of the dialkyl carbonate in the column bottom component ($B_B$) to be fed to the continuous multi-stage distillation column B2 is from 99.00 to 99.95% by mass, and
in the step (II), a purity of the dialkyl carbonate in the side-cut component ($B_S$) withdrawn from the side withdrawal port of the continuous multi-stage distillation column B2 is 99.99% by mass or more.

<2> The production method according to <1>, wherein the step (I) is performed in the presence of a compound containing Fe.
<3> The production method according to <2>, wherein a contacting surface area of the compound containing Fe with the low-purity dialkyl carbonate mixture ($A_T$) in the step (I) is $1.0 \times 10^{-3}$ $m^2 \cdot min/(kg/Hr)$ or more.
<4> The production method according to <2> or <3>, wherein the step (I) is performed in the presence of iron(II) oxide.
<5> The production method according to any of <1> to <4>, wherein a content of a high boiling point compound in the side-cut component ($B_S$) withdrawn from the side withdrawal port of the continuous multi-stage distillation column B2 is 30 mass ppm or less.
<6> The production method according to any of <1> to <5>, wherein a metal content in the side-cut component ($B_S$) withdrawn from the side withdrawal port of the continuous multi-stage distillation column B2 is 1 mass ppm or less.
<7> The production method according to any of <1> to <6>, wherein a water content in the side-cut component ($B_S$) withdrawn from the side withdrawal port of the continuous multi-stage distillation column B2 is 30 mass ppm or less.
<8> The production method according to any of <1> to <7>, wherein a total content of methanol and ethanol in the side-cut component ($B_S$) withdrawn from the side withdrawal port of the continuous multi-stage distillation column B2 is 20 mass ppm or less.
<9> The production method according to any one of <1> to <8>, wherein a content of 2-methoxy ethanol in the side-cut component ($B_S$) withdrawn from the side withdrawal port of the continuous multi-stage distillation column B2 is 50 mass ppm or less.
<10> The production method according to any one of <1> to <9>, wherein a temperature of the low-purity dialkyl carbonate mixture ($A_T$) to be fed to the continuous multi-stage distillation column B1 is set to 100 to 150°C.
<11> The production method according to <10>, wherein the low-purity dialkyl carbonate mixture ($A_T$) is heated with a heater, and a time required for feeding from the heater to the continuous multi-stage distillation column B1 is 5 minutes or less.
<12> The production method according to any of <1> to <11>, wherein the column top component ($B_T$) continuously withdrawn from the upper portion of the column of the continuous multi-stage distillation column B1 is condensed under conditions of a reflux drum temperature of 100 to 150°C.
<13> The production method according to any of <1> to <12>, wherein

the continuous multi-stage distillation column B2 comprises a column upper stage straight body portion, a column lower stage straight body portion having a diameter larger than that of the column upper stage straight body portion, and a taper portion connecting the column upper stage straight body portion to the column lower stage straight body portion, and
the side withdrawal port of the continuous multi-stage distillation column B2 is provided in the taper portion.

<14> The production method according to <13>, wherein, in the continuous multi-stage distillation column B2, a ratio of a column diameter $D_{21}$ (cm) of the column upper stage straight body portion to a column diameter $D_{22}$ (cm) of the column lower stage straight body portion satisfies the conditions of the following formula (ii):

$$0.2 < D_{21}/D_{22} < 1.0...(ii).$$

<15> The production method according to any of <1> to <14>, wherein, in the continuous multi-stage distillation column B1, a residence time of liquid in column calculated by the following formula (i) is 5 minutes or longer:

Residence time of liquid in column (min) = BTM volume (volume of liquid residing in the column BTM during operation (kg))/BTM withdrawal flow rate (flow rate for withdrawal of column bottom component (kg/min))...(i)

<16> The production method according to any of <1> to <15>, wherein, in the continuous multi-stage distillation column B2, the side-cut component ($B_s$) is withdrawn in a gaseous form.
<17> The production method according to any of <1> to <16>, wherein an internal of the continuous multi-stage distillation column B1 is a tray and/or a packing.
<18> The production method according to any of <1> to <17>, wherein a reflux ratio of the continuous multi-stage distillation column B1 is from 0.5 to 5.
<19> The production method according to any of <1> to <18>, wherein a reflux ratio of the continuous multi-stage distillation column B2 is from 0.2 to 4.
<20> The production method according to any of <1> to <19>, wherein, in the column bottom component ($B_B$) of the continuous multi-stage distillation column B1, a content of 2-methoxy ethanol in the dialkyl carbonate is 100 mass ppm or less.
<21> The production method according to any of <1> to <20>, wherein the column bottom component ($B_B$) of the continuous multi-stage distillation column B1 is fed directly to the continuous multi-stage distillation column B2, or fed to an industry-grade dialkyl carbonate tank and then fed from the tank to the continuous multi-stage distillation column B2.
<22> The production method according to any of <1> to <21>, wherein a content of a high boiling point compound in the column bottom component ($B_B$) of the continuous multi-stage distillation column B1 is 0.1 mass ppm or more.
<23> The production method according to any of <1> to <22>, comprising a step of reacting a cyclic carbonate with an aliphatic monohydric alcohol to provide a low-purity dialkyl carbonate mixture ($A_T$) containing a dialkyl carbonate.
<24> The production method according to any of <1> to <23>, comprising a step of continuously feeding a cyclic carbonate and an aliphatic monohydric alcohol into a continuous multi-stage distillation column A in which a catalyst is present, performing reaction and distillation simultaneously in the column, and continuously withdrawing a low-purity dialkyl carbonate mixture ($A_T$) containing a dialkyl carbonate generated and unreacted aliphatic monohydric alcohol from the upper portion of the column.
<25> A production apparatus for a dialkyl carbonate, comprising:

a continuous multi-stage distillation column B1, to which a low-purity dialkyl carbonate mixture ($A_T$) containing an dialkyl carbonate and an aliphatic monohydric alcohol is continuously fed, from an upper portion of which a column top component ($B_T$) containing the aliphatic monohydric alcohol as the main component is continuously withdrawn, and from a lower portion of which a column bottom component ($B_B$) containing the dialkyl carbonate as the main component is continuously withdrawn, and
a continuous multi-stage distillation column B2, to which a column bottom component ($B_B$) containing the dialkyl carbonate as the main component continuously withdrawn from column bottom portion of the continuous multi-stage distillation column B1 is continuously fed, the continuous multi-stage distillation column B2 having a side withdrawal port from which a side-cut component ($B_s$) containing the dialkyl carbonate as the main component is continuously withdrawn, wherein
a temperature of column bottom of the continuous multi-stage distillation column B1 can be set to 115°C or more, and
the production apparatus is configured so as to allow a compound containing Fe to exist in the continuous multi-stage distillation column B1.

<26> The production apparatus according to <25>, configured so as to enable iron(II) oxide to be fed in the continuous multi-stage distillation column B1.
<27> The production apparatus according to <25> or <26>, wherein a material in the continuous multi-stage distillation column B1 is carbon steel.
<28> The production apparatus according to any of <25> to <27>, comprising a heater that heats the low-purity dialkyl carbonate mixture ($A_T$) to be fed to the continuous multi-stage distillation column B1.
<29> The production apparatus according to any of <25> to <28>, wherein

the continuous multi-stage distillation column B2 comprises a column upper stage straight body portion, a column lower stage straight body portion having a diameter larger than that of the column upper stage straight body portion, and a taper portion connecting the column upper stage straight body portion to the column lower stage straight body portion, and
the side withdrawal port of the continuous multi-stage distillation column B2 is provided in the taper portion.

<30> The production apparatus according to <29>, wherein, in the continuous multi-stage distillation column B2, a ratio of a column diameter $D_{21}$ (cm) of the column upper stage straight body portion to a column diameter $D_{22}$ (cm) of the column lower stage straight body portion satisfies the conditions of the following formula (ii):

$$0.2 < D_{21}/D_{22} < 1.0...(ii).$$

<31> The production apparatus according to any of <25> to <30>, wherein an internal of the continuous multi-stage distillation column B1 is a tray and/or a packing.
<32> The production apparatus according to any of <25> to <31>, wherein the column bottom component ($B_B$) of the continuous multi-stage distillation column B1 is fed directly to the continuous multi-stage distillation column B2, or fed to an industry-grade dialkyl carbonate tank and then fed from the tank to the continuous multi-stage distillation column B2.

Advantageous Effects

The present invention can provide a production method of a dialkyl carbonate, which method reduces an amount of heat consumption in a separation and purification step of providing a high-purity dialkyl carbonate having a purity of 99.99% by mass or more from a dialkyl carbonate having a purity of 99.00 to 99.95% by mass, and an production apparatus for a dialkyl carbonate.

Brief Description of Drawings

[Figure 1] Figure 1 is a schematic view showing one exemplary flowchart of two separation and purification steps (I) and (II) in the production method of a dialkyl carbonate of the present invention.
[Figure 2] Figure 2 is a schematic view showing one exemplary flowchart of two separation and purification steps (I) and (II) in the production method of a dialkyl carbonate of the present invention.
[Figure 3] Figure 3 is a schematic view showing one exemplary continuous multi-stage distillation column.

Description of Embodiment

Hereinbelow, an embodiment of the present invention (hereinbelow, referred to as "the present embodiment") will be described in detail with reference to the drawings, if necessary, but the present invention is not limited to thereto and can be modified in various ways without departing from the spirit thereof.
The production method of a dialkyl carbonate of the present embodiment includes:

(I) a first separation and purification step (I) of continuously feeding a low-purity dialkyl carbonate mixture ($A_T$) containing a dialkyl carbonate and an aliphatic monohydric alcohol to a continuous multi-stage distillation column B1, continuously withdrawing a column top component ($B_T$) containing the aliphatic monohydric alcohol as the main component from the upper portion of the column, and continuously withdrawing a column bottom component ($B_B$)

containing the dialkyl carbonate as the main component from the lower portion of the column, and

(II) a second separation and purification step (II) of continuously feeding the column bottom component ($B_B$) containing the dialkyl carbonate as the main component continuously withdrawn from the column bottom portion of the continuous multi-stage distillation column B1 to a continuous multi-stage distillation column B2 having a side withdrawal port (hereinbelow, also referred to as "a side-cut withdrawal port"), and continuously withdrawing a side-cut component ($B_s$) containing the dialkyl carbonate as the main component from the side withdrawal port, in which method,

in the step (I), the concentration of the dialkyl carbonate in the low-purity dialkyl carbonate mixture ($A_T$) to be fed to the continuous multi-stage distillation column B1 is from 25.00 to 95.00% by mass, and the temperature of column bottom of the continuous multi-stage distillation column B1 is 115°C or more,
in the step (II), the concentration of the dialkyl carbonate in the column bottom component ($B_B$) to be fed to the continuous multi-stage distillation column B2 is from 99.00 to 99.95% by mass, and
in the step (II), the purity of the dialkyl carbonate in the side-cut component ($B_s$) withdrawn from the side withdrawal port of the continuous multi-stage distillation column B2 is 99.99% by mass or more.

The production method of a dialkyl carbonate of the present embodiment, as configured as above, can reduce the amount of heat consumption in a separation and purification step of providing a high-purity dialkyl carbonate having a purity of 99.99% by mass or more from a dialkyl carbonate having a purity of 99.00 to 99.95% by mass. For example, as separation and purification can be achieved at a small reflux ratio, it is possible to reduce the amount of heat consumption.
Usually, in order to enhance the purity of a dialkyl carbonate to be ultimately provided, the concentration of the dialkyl carbonate in the low-purity dialkyl carbonate mixture ($A_T$) to be fed to the continuous multi-stage distillation column B1 should be set to high in advance (e.g., a purity of 99.95% by mass as described in Patent Literatures 1 and 2). However, it has been found that, in the production method of a dialkyl carbonate of the present embodiment, surprisingly, a high-purity dialkyl carbonate having a purity of 99.99% by mass or more can be produced from a dialkyl carbonate having a purity of 99.00 to 99.95% by mass with a reduced amount of heat consumption by setting the concentration of the dialkyl carbonate within a range lower than before as mentioned above.
The mechanism to develop such an effect is not clear, but the present inventor considers as follows. The low-purity dialkyl carbonate mixture ($A_T$) to be fed to the continuous multi-stage distillation column B1 may usually contain an aliphatic monohydric alcohol, a trace amount of an alkoxy alcohol, a trace amount of an aliphatic carbonate ether, and the like, as impurities. 2ME is difficult to separate by distillation from dialkyl carbonates, and thus highly purifying a dialkyl carbonate containing such impurities to a purity of 99.99% by mass or more requires a large amount of heat consumption, that is, a large reflux ratio. In the step (I) of the production method of a dialkyl carbonate of the present embodiment, the concentration of the dialkyl carbonate in the low-purity dialkyl carbonate mixture ($A_T$) to be fed to the continuous multi-stage distillation column B1 is set within a low range such as 25.00 to 95.00% by mass, and the temperature of column bottom for separation by distillation of the aliphatic monohydric alcohol and dialkyl carbonate is set to a high temperature (e.g., 115°C or more). Lowering the concentration of the dialkyl carbonate in the feed mixture and raising the temperature of column bottom enables 2ME, which is a substance inhibiting high purification of the dialkyl carbonate, to be converted into a high boiling point compound. A high boiling point compound is easily separated by distillation from dialkyl carbonates. Thus, in the continuous multi-stage distillation column B2 of the step (II), it is considered that the dialkyl carbonate can be highly purified to a purity of 99.99% by mass or more with a lowered reflux ratio and a reduced amount of heat consumption.
The upper limit of the purity of the dialkyl carbonate in the side-cut component ($B_s$) is, but is not limited to, 99.999% by mass, for example.
In the present embodiment, the main component means a component of which the mass proportion is the highest, being a component of 30% by mass or more, preferably 40% by mass or more, more preferably 50% by mass or more.
In the production method of the present embodiment, two separation and purification steps (I) and (II) are performed to thereby allow a high-purity dialkyl carbonate having a purity of 99.99% by mass or more to be separated.
In the production method of the present embodiment, the two separation and purification steps (I) and (II) preferably include, for example, as shown in Figure 1, a first separation and purification step (I) of continuously feeding a low-purity dialkyl carbonate mixture ($A_T$) containing a dialkyl carbonate and an aliphatic monohydric alcohol to a first continuous multi-stage distillation column B1, continuously withdrawing a column top component ($B_T$) containing the aliphatic monohydric alcohol as the main component from the upper portion of the column, and continuously withdrawing a column bottom component ($B_B$) containing the dialkyl carbonate as the main component from the lower portion of the column, and a second separation and purification step (II) of continuously feeding the column bottom component ($B_B$) containing the dialkyl carbonate as the main component continuously withdrawn from the column bottom portion of the

continuous multi-stage distillation column B1 to a second continuous multi-stage distillation column B2 having a side withdrawal port, continuously withdrawing a column top component ($B_t$), which is a low boiling point component, from the upper portion of the column, continuously withdrawing a side-cut component ($B_s$) containing the dialkyl carbonate as the main component from the side withdrawal port, and continuously withdrawing, from the column bottom, a column bottom component ($B_b$), which is a high boiling point component, from the lower portion of the column.

[First separation and purification step (I)]

In the production method of a dialkyl carbonate of the present embodiment, the step (I) is a first separation and purification step of continuously feeding a low-purity dialkyl carbonate mixture ($A_T$) containing a dialkyl carbonate and an aliphatic monohydric alcohol to a continuous multi-stage distillation column B1, continuously withdrawing a column top component ($B_T$) containing the aliphatic monohydric alcohol as the main component from the upper portion of the column, and continuously withdrawing a column bottom component ($B_B$) containing the dialkyl carbonate as the main component from the lower portion of the column.

Hereinbelow, the step (I) will be described in detail.

In the step (I), the concentration of the dialkyl carbonate in the low-purity dialkyl carbonate mixture ($A_T$) to be fed to the continuous multi-stage distillation column B1 is from 25.00 to 95.00% by mass, preferably from 30.00 to 90.00% by mass, more preferably from 35.00 to 85.00% by mass. The concentration of the dialkyl carbonate in the low-purity dialkyl carbonate mixture ($A_T$) means the purity of the dialkyl carbonate of the low-purity dialkyl carbonate mixture ($A_T$).

Examples of the dialkyl carbonate include, but are not particularly limited to, dimethyl carbonate and diethyl carbonate. Of these, dimethyl carbonate is preferred.

Examples of the aliphatic monohydric alcohol include, but are not particularly limited to, methanol and ethanol. Of these, methanol is preferred.

The low-purity dialkyl carbonate mixture ($A_T$) may contain, as components other than the dialkyl carbonate and aliphatic monohydric alcohol, for example, an alkoxy alcohol, an aliphatic carbonate ether, an alkylene oxide, or carbon dioxide, but not particularly limited thereto. Examples of the alkoxy alcohol include, but are not particularly limited to, 2-methoxy ethanol (hereinbelow, also denoted as "2ME"). Examples of the aliphatic carbonate ether include, but are not particularly limited to, ethylene glycol monomethyl carbonate (hereinbelow, also denoted as "EMMC"). Examples of the alkylene oxide include, but are not particularly limited to, ethylene oxide.

The concentration of the aliphatic monohydric alcohol in the low-purity dialkyl carbonate mixture ($A_T$) is preferably from 5.00 to 75.00% by mass, more preferably from 10.00 to 70.00% by mass, still more preferably from 15.00 to 65.00% by mass.

The concentration of 2-methoxy ethanol in the low-purity dialkyl carbonate mixture ($A_T$) is preferably from 0.00 to 1.00% by mass, more preferably from 0.00 to 0.80% by mass, still more preferably from 0.00 to 0.60% by mass.

The content of carbon dioxide in low-purity dialkyl carbonate mixture ($A_T$) is preferably from 0.00 to 1.00% by mass, more preferably from 0.00 to 0.50% by mass, still more preferably from 0.00 to 0.10% by mass.

The temperature of column bottom of the continuous multi-stage distillation column B1 is 115°C or more, preferably from 140 to 250°C, more preferably from 180 to 220°C. When the temperature of column bottom of the continuous multi-stage distillation column B1 is in the range, 2ME, which is a substance inhibiting high purification of the dialkyl carbonate, can be converted into a high boiling point compound, and the amount of heat consumption can be reduced in the continuous multi-stage distillation column B2 of the step (II).

In the continuous multi-stage distillation column B1, the residence time of liquid in column calculated by the following formula (i) is preferably 5 minutes or longer, more preferably from 10 to 150 minutes, still more preferably from 15 to 120 minutes. When the residence time of liquid in column is in the range in the continuous multi-stage distillation column B1, the conversion of 2ME increases, and the concentration of 2ME remaining in the column bottom component ($B_B$) to be withdrawn from the continuous multi-stage distillation column B1 tends to decrease (e.g., 10 mass ppm or less). Residence time of liquid in column (min) = BTM volume (volume of liquid residing in the column BTM during operation (kg))/BTM withdrawal flow rate (flow rate for withdrawal of column bottom component (kg/min))...(i)

The step (I) is preferably performed in the presence of a compound containing Fe.

For example, a compound containing Fe preferably exists inside the continuous multi-stage distillation column B1 or on the surface thereof. When the step (I) is performed in the presence of a compound containing Fe, a reaction to convert 2ME, which is a substance inhibiting high purification of the dialkyl carbonate, into a high boiling point compound can be further facilitated. Consequently, the final purity of the dialkyl carbonate is further easily allowed to be 99.99% by mass or more, for example, and additionally, production of such a dialkyl carbonate having a high purity of 99.99% by mass or more tends to be enabled with a further smaller amount of vapor, that is, at a smaller reflux ratio.

Examples of the compound containing Fe include, but are particularly limited to, iron oxide. Examples of iron oxide include iron(II) oxide.

The surface area contacting between the low-purity dialkyl carbonate mixture ($A_T$) and the compound containing

Fe is preferably made larger, from the viewpoint of further facilitating the reaction to convert 2ME into a high boiling point compound. More specifically, the contacting surface area of the compound containing Fe with the low-purity dialkyl carbonate mixture ($A_T$) in the step (I) is preferably 1.0 × $10^{-3}$ $m^2$·min/(kg/Hr) or more. The contacting surface area is preferably 1.5 × $10^{-3}$ $m^2$·min/(kg/Hr) or more, more preferably 2.0 × $10^{-3}$ $m^2$·min/(kg/Hr) or more. The upper limit of the contacting surface area is, but is not limited to, 10,000 × $10^{-3}$ $m^2$·min/(kg/Hr) or less, for example.

The contacting surface area, when a powder of a compound containing Fe is added, can be determined by the following formula.

Contacting surface area ($m^2$·min/(kg/Hr)) = [(surface area in average particle size of powder added) ($m^2$) × residence time of liquid in column (min)]/feed flow rate (kg/hr)

The above residence time of liquid in column can be determined by the following formula.

Residence time of liquid in column (min) = BTM volume (volume of liquid residing in the column BTM during operation (kg))/BTM withdrawal flow rate (flow rate for withdrawal of column bottom component (kg/min))

The contacting surface area, when a compound containing Fe is contained in distillation column material such as inner wall, can be determined by the following formula.

contacting surface area ($m^2$·min/(kg/Hr)) = [(area in column at which BTM volume is in contact with liquid ($m^2$) × (residence time in column (min))]/(feed flow rate (kg/hr))

The material inside the distillation column B1 is carbon steel. Iron(II) oxide can be generated on the surface by inflowing oxygen when the operation is stopped or the apparatus is assembled. Although carbon steel is not usually used for purification of a high-purity material, in the present embodiment, use of carbon steel as the material inside the distillation column B1 enables the amount of heat consumption to be reduced in separation and purification of a high-purity dialkyl carbonate in the distillation column B2. Examples of the material inside the distillation column B1 include the inner wall surface of the distillation column B1.

Internals of each of the recovery section and the concentration section of the continuous multi-stage distillation column B1 are preferably trays and/or packings, more preferably trays. The type of the trays is not limited, and a bubble-cap tray, a sieve tray, a ripple tray, a ballast tray, a valve tray, a counterflow tray, a Unifrax tray, a Superfrac tray, a Maxfrac tray, a dual flow tray, a grid plate tray, a turbogrid plate tray, a Kittel tray, and the like are preferred. In the case in which, in this continuous multi-stage distillation column, the amount of 2ME present is small and there are stages in which no reaction substantially takes place (e.g., stages above the stage at which a feed liquid is introduced), a distillation column packed with this stage packing, i.e. a multi-stage distillation column having both a tray portion and a portion packed with packings, is also preferred. Such packings are not particularly limited, and irregular packings such as a Raschig ring, a Lessing ring, a Pall ring, a Berl saddle, an Intalox saddle, a Dixon packing, a McMahon packing, or Heli-Pak, and regular packings such as Mellapak, Gempak, Techno-pack, Flexipac, a Sulzer packing, a Goodroll packing, or Glitschgrid are preferred. When the internals of each of the recovery section and the concentration section of the continuous multi-stage distillation column B1 are trays and/or packings, the final purity of the dialkyl carbonate is further

easily allowed to reach a high purity (a purity of 99.99% by mass or more) of such a level as to enable the dialkyl carbonate to be used for lithium ion battery electrolyte solutions, for example. Additionally, production of such a high-purity (a purity of 99.99% by mass or more) dialkyl carbonate from an industry-grade dialkyl carbonate tends to be enabled with a further reduced amount of heat fed (at a smaller reflux ratio).

The reflux ratio of the continuous multi-stage distillation column B1 is preferably from 0.5 to 20, more preferably from 0.8 to 15, still more preferably from 1 to 5. In the production method of a dialkyl carbonate of the present embodiment, making the reflux ratio small as above enables the reaction of converting 2ME into a high boiling point compound to be further facilitated to thereby enable the amount of heat consumption to be reduced in the subsequent second separation and purification step (II).

In the column bottom component ($B_B$) of the continuous multi-stage distillation column B1, the concentration of the dialkyl carbonate is from 99.00 to 99.95% by mass. In the column bottom component ($B_B$) of the continuous multi-stage distillation column B1, the content of 2-methoxy ethanol is preferably 100 mass ppm or less, more preferably 50 mass ppm or less, still more preferably 10 mass ppm or less. In the column bottom component ($B_B$) of the continuous multi-stage distillation column B1, the lower limit of the content of 2-methoxy ethanol is, but is not limited to, 0 mass ppm, for example. When the purity of the dialkyl carbonate and the content of 2-methoxy ethanol in the column bottom component ($B_B$) of the continuous multi-stage distillation column B1 are in the ranges, the final purity of the dialkyl carbonate is further easily allowed to reach a high purity (a purity of 99.99% by mass or more) of such a level as to enable the dialkyl carbonate to be used for lithium ion battery electrolyte solutions, for example. Additionally, production of such a high-purity (a purity of 99.99% by mass or more) dialkyl carbonate from an industry-grade dialkyl carbonate tends to be enabled with a further smaller amount of heat consumption (at a smaller reflux ratio).

The content of the high boiling point compound in the column bottom component ($B_B$) of the continuous multi-stage distillation column B1 is preferably 0.1 mass ppm or more, more preferably 1 mass ppm or more, still more preferably 100 mass ppm or more. As the high boiling point compound is considered to be a substance converted from 2-methoxy ethanol, which is difficult to separate from the dialkyl carbonate in distillation, the content of the high boiling point compound may be in the range mentioned above. In other words, when the content of the high boiling point compound in the column bottom component ($B_B$) of the continuous multi-stage distillation column B1 is in the range, the final purity of the dialkyl carbonate is further easily allowed to reach a high purity (a purity of 99.99% by mass or more) of such a level as to enable the dialkyl carbonate to be used for lithium ion battery electrolyte solutions, for example. Additionally, production of such a high-purity (a purity of 99.99% by mass or more) dialkyl carbonate from an industry-grade dialkyl carbonate tends to be enabled with a further smaller amount of heat consumption (at a smaller reflux ratio).

In the column bottom component ($B_B$) of the continuous multi-stage distillation column B1, the upper limit of the content of the high boiling point compound is, but is not limited to, 1.0% by mass, for example.

In the present embodiment, a "high boiling point compound" means a compound having a boiling point higher by 100°C or more than the boiling point of the dialkyl carbonate which is the main component under a pressure of 760 mmHg. Here the "main component" means a component of which the proportion is the highest of the dialkyl carbonates contained in the column bottom component ($B_B$) of the continuous multi-stage distillation column B1.

In the present embodiment, the concentration of the dialkyl carbonate and content of each component can be measured by the methods described in Examples mentioned below.

In the step (I), a continuous multi-stage distillation column B1 is used to separate by distillation a low-purity dialkyl carbonate mixture ($A_T$) containing a dialkyl carbonate and an aliphatic monohydric alcohol into a column top component ($B_T$) containing the aliphatic monohydric alcohol as the main component and a column bottom component ($B_B$) containing the dialkyl carbonate as the main component.

The continuous multi-stage distillation column B1 according to the step (I) preferably has a function of stably separating the dialkyl carbonate from a large amount of the low-purity dialkyl carbonate mixture ($A_T$) at predetermined separation efficiency over a long period and preferably satisfies various conditions simultaneously therefor.

In the present embodiment, the low-purity dialkyl carbonate mixture ($A_T$) of the dialkyl carbonate and the aliphatic monohydric alcohol is continuously fed to the continuous multi-stage distillation column B1, the column top component ($B_T$) containing the aliphatic monohydric alcohol as the main component is continuously withdrawn (preferably in a gaseous form) from the upper portion of the column, and the column bottom component ($B_B$) containing the dialkyl carbonate as the main component is continuously withdrawn (preferably in a liquid form) from the lower portion of the column. The low-purity dialkyl carbonate mixture ($A_T$), when fed into the continuous multi-stage distillation column B1, may be fed in a gaseous form or may be fed in a liquid form. In advance of feeding the low-purity dialkyl carbonate mixture ($A_T$) into the continuous multi-stage distillation column B1, the mixture ($A_T$) is also preferably heated or cooled in order to make the temperature thereof close to the liquid temperature in the vicinity of the feed port of the distillation column B1.

The position at which the low-purity dialkyl carbonate mixture ($A_T$) is fed into the continuous multi-stage distillation column B1 is preferably in the vicinity between the recovery section and the concentration section. The continuous multi-stage distillation column B1 preferably has a reboiler for heating the distillate and a reflux device.

In the present embodiment, the low-purity dialkyl carbonate mixture ($A_T$) is preferably fed into the continuous multi-stage distillation column B1 at about 2 ton/hr or more and separated by distillation, and the column top component ($B_T$), which is a low boiling point mixture, is withdrawn from the upper portion of the distillation column B1, and the column bottom component ($B_B$), which is a high boiling point mixture, is withdrawn from the lower portion thereof.

In the step (I), the concentration of aliphatic monohydric alcohols in the column top component ($B_T$), which is a low boiling point mixture, can be set to preferably 40% by mass or more, more preferably 45% by mass or more, still more preferably 50% by mass or more. The upper limit of the concentration of the aliphatic monohydric alcohols in the column top component ($B_T$) is, but is not particularly limited to, 100% by mass, for example. The amount of alcohols to be separated as the main component of the column top component ($B_T$), which is a low boiling point mixture, is preferably 300 kg/hr or more, preferably 350 kg/hr or more, more preferably 400 kg/hr or more. Other components of the column top component ($B_T$), which are a low boiling point mixture, are mainly dialkyl carbonates. Thus, the components, as they are or as admixture with alcohols recovered in other steps, can be reused as an aliphatic monohydric alcohol to be reacted with a cyclic carbonate. This is one of preferable aspects of the present embodiment. When the amount of alcohols recovered is insufficient, an aliphatic monohydric alcohol is newly added.

The column bottom component ($B_B$), which is a high boiling temperature mixture, to be separated in the step (I) contains a dialkyl carbonate as the main component, and the content of unreacted aliphatic monohydric alcohol is preferably 1% by mass or less, more preferably 0.8% by mass or less, still more preferably 0.6% by mass or less. The lower limit of the content of the unreacted aliphatic monohydric alcohol in the column bottom component ($B_B$) is, but is not particularly limited to, 0% by mass, for example.

In a preferable aspect of the present embodiment, the reaction is performed using halogen-free starting materials and catalysts, and thus it is possible to make a dialkyl carbonate to be generated contain no halogen. Accordingly, in the present embodiment, the halogen content is preferably 0.1 mass ppm or less, more preferably 1 mass ppb or less (beyond the detection limit of ion chromatography).

The pressure of column bottom in the continuous multi-stage distillation column B1 in the step (I) may vary depending on the composition inside the column and the temperature of column bottom to be used but is preferably from 0.1 MPaG to 3.0 MPaG, more preferably from 0.15 MPaG to 2.5 MPaG, still more preferably from 0.2 MPaG to 2.0 MPaG.

Materials constituting the continuous multi-stage distillation column B1 used in the step (I) are, but are not particularly limited to, preferably metal materials such as carbon steel and stainless steel, for example, in respect of the quality of dialkyl carbonates and diols to be produced and separated.

[Step (II)]

In the production method of a dialkyl carbonate of the present embodiment, the step (II) is a second separation and purification step of continuously feeding the column bottom component ($B_B$) containing the dialkyl carbonate as the main component continuously withdrawn from the column bottom portion of the continuous multi-stage distillation column B1 of the step (I) mentioned above to the continuous multi-stage distillation column B2 having a side withdrawal port and continuously withdrawing a side-cut component ($B_s$) containing the dialkyl carbonate as the main component from the side withdrawal port.

In the second separation and purification step, the column top component ($B_t$), which is a low boiling point component, may be continuously withdrawn from the upper portion of the column. Also in the second separation and purification step, the column bottom component ($B_b$), which is a high boiling point component, may be, from the column bottom, continuously withdrawn from the lower portion of the column.

Hereinbelow, the step (II) will be described in detail.

In the step (II), the concentration of the dialkyl carbonate in the column bottom component ($B_B$) to be fed to the continuous multi-stage distillation column B2 is from 99.00 to 99.95% by mass, preferably from 99.2 to 99.95% by mass, more preferably from 99.4 to 99.95% by mass. When the concentration of the dialkyl carbonate in the column bottom component ($B_B$) of the continuous multi-stage distillation column B1 is in the range, the final purity of the dialkyl carbonate is easily allowed to reach a high purity of 99.99% by mass or more, for example, which purity is of such a level as to enable the dialkyl carbonate to be used for lithium ion battery electrolyte solutions. Additionally, production of such a high-purity dialkyl carbonate having a purity of 99.99% by mass or more from an industry-grade dialkyl carbonate is enabled with a small amount of heat consumption, that is, at a small reflux ratio.

In the step (II), the purity of the dialkyl carbonate in the side-cut component ($B_s$) withdrawn from the side withdrawal port of the continuous multi-stage distillation column B2 is 99.99% by mass or more. Such a high-purity dialkyl carbonate can be used for lithium ion battery electrolyte solutions, for example.

The temperature of column bottom of the continuous multi-stage distillation column B2 is preferably 120°C or less, more preferably from 60 to 110°C, still more preferably from 65 to 105°C. When the temperature of column bottom of the continuous multi-stage distillation column B2 is in the range, heating can be made with vapor that may be generated

by heat exchange of a condenser in the upper portion of the column of the multi-stage distillation column B1, and a high-purity dialkyl carbonate having a purity of 99.99% by mass or more of such a level as to enable the dialkyl carbonate to be used for lithium ion battery electrolyte solutions can be produced from an industry-grade dialkyl carbonate having a purity of 99.0% by mass or more, for example, with a more reduced amount of heat fed from the outside.

The content of the high boiling point compound in the side-cut component ($B_s$) withdrawn from the side withdrawal port of the continuous multi-stage distillation column B2 is preferably 30 mass ppm or less, more preferably 25 mass ppm or less, still more preferably 20 mass ppm or less. For example, when purification is made only with the continuous multi-stage distillation column B1 such that the purity of the dialkyl carbonate finally provided is 99.99% by mass or more followed by withdrawal from the column bottom, it is revealed that a high boiling point compound is contained in the dialkyl carbonate. In the case of use for electrolyte solution applications for lithium ion batteries or the like, the high boiling point compound leads to a decrease in the performance and thus is desirably removed. According to the production method of a dialkyl carbonate according to the present embodiment, it is possible to lower the content of the high boiling point compound while suppressing the amount of heat consumption through the two-stage steps: the step (I) and step (II). Here, the definition of the "high boiling point compound" is as mentioned above.

The metal content in the side-cut component ($B_s$) withdrawn from the side withdrawal port of the continuous multi-stage distillation column B2 is preferably 1 mass ppm or less, more preferably 0.8 mass ppm or less, still more preferably 0.6 mass ppm or less. For example, when purification is made only with the continuous multi-stage distillation column B1 such that the purity of the dialkyl carbonate finally provided is 99.99% by mass or more followed by withdrawal from the column bottom, it is revealed that metal is contained in the dialkyl carbonate. In the case of use for electrolyte solution applications for lithium ion batteries or the like, the metal leads to a decrease in the performance and thus is desirably removed. According to the production method of a dialkyl carbonate according to the present embodiment, it is possible to lower the content of the metal while suppressing the amount of heat consumption through the two-stage steps: the step (I) and step (II).

The water content in the side-cut component ($B_s$) withdrawn from the side withdrawal port of the continuous multi-stage distillation column B2 is preferably 30 mass ppm or less, more preferably 25 mass ppm or less, still more preferably 20 mass ppm or less. In the case of use for electrolyte solution applications for lithium ion batteries or the like, moisture leads to a decrease in the performance and thus is desirably removed. According to the production method of a dialkyl carbonate according to the present embodiment, it is possible to lower the content of the moisture while suppressing the amount of heat consumption through the two-stage steps: the step (I) and step (II).

The total content of methanol and ethanol in the side-cut component ($B_s$) withdrawn from the side withdrawal port of the continuous multi-stage distillation column B2 is preferably 20 mass ppm or less. A high-purity dialkyl carbonate having a content of such components within the range is extremely useful for lithium ion battery electrolyte solutions, for example.

The 2ME content in the side-cut component ($B_s$) withdrawn from the side withdrawal port of the continuous multi-stage distillation column B2 is preferably 50 mass ppm or less, more preferably 40 mass ppm or less, still more preferably 30 mass ppm or less. In the case of use for electrolyte solution applications for lithium ion batteries or the like, 2ME leads to a decrease in the performance and thus is desirably removed. According to the production method of a dialkyl carbonate according to the present embodiment, 2ME is converted into a high boiling point compound or the like and sufficiently removed in the step (I) and then further purified by the step (II) by undergoing the two-stage steps: the step (I) and step (II). Thus, it is possible to lower the content of 2ME while suppressing the amount of heat consumption. The lower limit of the content of each of the high boiling point compound, 2ME, metal, water, methanol, and ethanol in the side-cut component ($B_s$) is, but is not particularly limited to, 0 mass ppm, for example.

In the continuous multi-stage distillation column B2, the side-cut component ($B_s$) is preferably withdrawn in a gaseous form. When the side-cut component ($B_s$) is withdrawn in a gaseous form in the continuous multi-stage distillation column B2, the amount of the high boiling component and the metal content with respect to the dialkyl carbonate tend to be suppressed in the side-cut component ($B_s$).

The reflux ratio of the continuous multi-stage distillation column B2 is preferably from 0.2 to 4, more preferably from 0.6 to 2.0, still more preferably from 0.8 to 1.5. In the production method of a dialkyl carbonate of the present embodiment, when the reflux ratio is made small as above, the amount of heat consumption can be reduced, and additionally, the final purity of the dialkyl carbonate is enabled to reach a high purity of 99.99% by mass or more, for example, which purity is of such a level as to enable the dialkyl carbonate to be used for lithium ion battery electrolyte solutions.

In the continuous multi-stage distillation column B2, the ratio of the column diameter $D_{21}$ (cm) above the side withdrawal port to the column diameter $D_{22}$ (cm) below the side withdrawal port preferably satisfies the conditions of the following formula (ii). When $D_{21}/D_{22}$ satisfies the conditions of the following formula (ii), production of a high-purity dialkyl carbonate having a purity of 99.99% by mass or more, which purity is of such a level as to enable the dialkyl carbonate to be used for lithium ion battery electrolyte solutions, tends to be enabled with a further smaller amount of heat consumption, that is, at a further smaller reflux ratio.

$$0.2 < D_{21}/D_{22} < 1.0 \ldots (ii)$$

From the similar viewpoint, $D_{21}/D_{22}$ is more preferably from 0.3 to 1.0.

In the step (II), the continuous multi-stage distillation column B2 is used for separating by distillation the side-cut component ($B_s$) containing the dialkyl carbonate as the main component from the column bottom component ($B_B$) containing the dialkyl carbonate as the main component continuously withdrawn from the column bottom portion of the continuous multi-stage distillation column B1. The column top component ($B_t$), which is a low boiling point component, and the column bottom component ($B_b$), which is a high boiling point component, may be further separated by distillation from the column bottom component ($B_B$) with the continuous multi-stage distillation column B2.

The recovery section and the concentration section of the continuous multi-stage distillation column B2 in the step (II) are each preferably a distillation column having the trays and/or packings as internals. A multi-stage distillation column having a tray portion and a portion packed with packings in combination also can be used.

The theoretical number of stages of the internals of both the recovery section and the concentration section of the continuous multi-stage distillation column B2 is preferably from 3 to 40.

The internal referred to in the present embodiment means an area in the distillation column at which gas and liquid are actually brought into contact with each other. Preferred examples of such trays include, but are not particularly limited to, a bubble-cap tray, a sieve tray, a ripple tray, a ballast tray, a valve tray, a counterflow tray, a Unifrax tray, a Superfrac tray, a Maxfrac tray, a dual flow tray, a grid plate tray, a turbogrid plate tray, and a Kittel tray. Preferred examples of packings include, but are not particularly limited to, irregular packings such as a Raschig ring, a Lessing ring, a Pall ring, a Berl saddle, an Intalox saddle, a Dixon packing, a McMahon packing, or Heli-Pak, and regular packings such as Mellapak, Gempak, Techno-pack, Flexipac, a Sulzer packing, a Goodroll packing, or Glitschgrid.

In the present embodiment, it is preferred that the column bottom component ($B_B$) containing the dialkyl carbonate as the main component continuously withdrawn from the column bottom portion of the continuous multi-stage distillation column B1 be continuously fed to the continuous multi-stage distillation column B2, the column top component ($B_t$), which is a low boiling point component, be continuously withdrawn from the upper portion of the column, the side-cut component ($B_s$) containing the dialkyl carbonate as the main component be continuously withdrawn from the side withdrawal port, and the column bottom component ($B_b$), which is a high boiling point component, from the column bottom, be continuously withdrawn from the lower portion of the column. In advance of feeding the column bottom component ($B_B$) to the continuous multi-stage distillation column B2, the component ($B_B$) is also preferably heated or cooled in order to make the temperature thereof close to the liquid temperature in the vicinity of the feed port of the distillation column B2.

The position at which the column bottom component ($B_B$) is fed into the continuous multi-stage distillation column B2 is preferably in the vicinity between the recovery section and the concentration section. The continuous multi-stage distillation column B2 preferably has a reboiler for heating the distillate and a reflux device.

In the present embodiment, the column bottom component ($B_B$) is preferably withdrawn from the continuous multi-stage distillation column B1 at about 2 ton/hr or more, fed into the continuous multi-stage distillation column B2, and separated by distillation. The column top component ($B_t$), which is a low boiling point component, is continuously withdrawn from the upper portion of the distillation column B2, and the column bottom component ($B_b$), which is a high boiling point component, is continuously withdrawn from the lower portion of the distillation column B2.

Other components of the column top component ($B_t$), which is a low boiling point component, are mainly dialkyl carbonates. Thus the components, as they are or as admixture with alcohols recovered in other steps, can be reused as aliphatic monohydric alcohols to be reacted with a cyclic carbonate. This is one of preferable aspects of the present embodiment. When the amount of the alcohols recovered is insufficient, an aliphatic monohydric alcohol is preferably newly added.

The column bottom component ($B_B$) of the continuous multi-stage distillation column B1 is preferably fed directly to the continuous multi-stage distillation column B2, or fed to an industry-grade dialkyl carbonate tank and then fed from the tank to the continuous multi-stage distillation column B2.

The low-purity dialkyl carbonate mixture ($A_T$) containing a dialkyl carbonate and an aliphatic monohydric alcohol used in the step (I) is not particularly limited, may be, for example, a mixture obtained by subjecting a cyclic carbonate and an aliphatic monohydric alcohol to reaction and distillation simultaneously in the continuous multi-stage distillation column in which a catalyst is present, may be a commercially available dialkyl carbonate, or may be a component separated from the distillation column of the dialkyl carbonate production step.

[Step ($\alpha$)]

The production method of a dialkyl carbonate of the present embodiment preferably includes a step ($\alpha$) of reacting a cyclic carbonate with an aliphatic monohydric alcohol to provide a low-purity dialkyl carbonate mixture ($A_T$)

containing a dialkyl carbonate.

The step (α) is also preferably a step of continuously feeding a cyclic carbonate and an aliphatic monohydric alcohol into a continuous multi-stage distillation column A in which a catalyst is present, performing reaction and distillation simultaneously in the column, and continuously withdrawing a low-purity dialkyl carbonate mixture ($A_T$) containing a dialkyl carbonate generated and unreacted aliphatic monohydric alcohol from the upper portion of the column.

Further, the step (α) is more preferably a step of continuously producing a dialkyl carbonate and a diol by a reactive distillation method of continuously withdrawing a high boiling temperature reaction mixture ($A_B$) containing the diol from the lower portion of the column.

Hereinbelow, the step (α) will be described in detail.

The reaction in the step (α) is a reversible, equilibrium transesterification reaction represented by the following formulas, in which a dialkyl carbonate (C) and a diol (D) are produced from a cyclic carbonate (A) and an aliphatic monohydric alcohol (B):

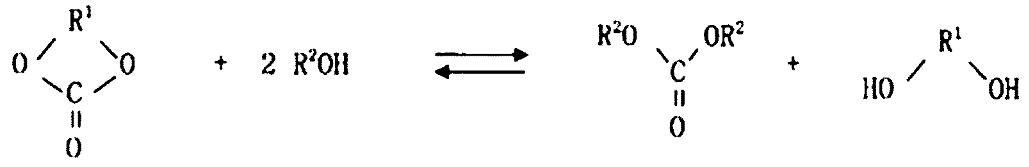

(A) (B) (C) (D)

wherein, $R^1$ represents a divalent group $-(CH_2)_m-$, wherein m is an integer of 2 to 6, one or more hydrogen of which may be substituted with an alkyl group having 1 to 10 carbon atoms or an aryl group, and $R^2$ represents a monovalent aliphatic group having 1 to 12 carbon atoms, one or more hydrogen of which may be substituted with an alkyl group having 1 to 10 carbon atoms or an aryl group.

A cyclic carbonate used as a starting material in the step (α) is a compound represented by (A) in the above formula and is not particularly limited. For example, alkylene carbonates, such as ethylene carbonate and propylene carbonate, 1,3-dioxacyclohexa-2-one, 1,3-dioxacyclohepta-2-one, and the like are preferably used. Ethylene carbonate and propylene carbonate are more preferably used because of their good availability, and ethylene carbonate is particularly preferably used.

An aliphatic monohydric alcohol as the other starting material is a compound which is represented by (B) in the above formulas, and an aliphatic monohydric alcohol having a boiling point lower than that of a generating diol is preferably used. Accordingly, specific examples of the aliphatic monohydric alcohol, although may depend on the type of the cyclic carbonate used, include methanol, ethanol, propanol (each of the isomers), allyl alcohol, butanol (each of the isomers), 3-buten-1-ol, amyl alcohol (each of the isomers), hexyl alcohol (each of the isomers), heptyl alcohol (each of the isomers), octyl alcohol (each of the isomers), nonyl alcohol (each of the isomers), decyl alcohol (each of the isomers), undecyl alcohol (each of the isomers), dodecyl alcohol (each of the isomers), cyclopentanol, cyclohexanol, cycloheptanol, cyclooctanol, methylcyclopentanol (each of the isomers), ethylcyclopentanol (each of the isomers), methylcyclohexanol (each of the isomers), ethylcyclohexanol (each of the isomers), dimethylcyclohexanol (each of the isomers), diethylcyclohexanol (each of the isomers), phenylcyclohexanol (each of the isomers), benzyl alcohol, phenethyl alcohol (each of the isomers), and phenylpropanol (each of the isomers). Further, these aliphatic monohydric alcohols may be substituted with a substituent, such as a halogen, a lower alkoxy group, a cyano group, an alkoxycarbonyl group, an aryloxycarbonyl group, an acyloxy group, or a nitro group.

Of these aliphatic monohydric alcohols, alcohols having 1 to 6 carbon atoms are preferably used, and alcohols having 1 to 4 carbon atoms such as methanol, ethanol, propanol (each of the isomers), and butanol (each of the isomers) are further preferred. When ethylene carbonate or propylene carbonate is used as a cyclic carbonate, methanol and ethanol are preferred, and particularly preferred is methanol.

In the step (α), a catalyst is caused to be present in the reactive distillation column A. The method for causing a catalyst to be present may be any method and is not particularly limited. For example, in the case of a homogeneous catalyst soluble in the reaction liquid under the reaction conditions, the catalyst is continuously fed into the reactive distillation column to thereby enable the catalyst to be present in the liquid phase in the reactive distillation column. Alternatively, in the case of a heterogeneous catalyst insoluble in the reaction liquid under the reaction conditions, a solid catalyst is placed in the reactive distillation column to thereby enable the catalyst to be present in the reaction system, or the method may combine these.

When a homogeneous catalyst is continuously fed into the reactive distillation column, the catalyst may be fed together with a cyclic carbonate and/or an aliphatic monohydric alcohol, or may be fed to a position different from that at which the starting material is fed. Since the region where the reaction actually takes place in the distillation column

is below the position at which the homogeneous catalyst is fed, the catalyst is preferably fed to the region between the top of the column and the position at which the starting material is fed. The number of stages in which the catalyst is present is preferably 5 or more, more preferably 7 or more, and still more preferably 10 or more.

When a heterogeneous solid catalyst is used, the number of stages in which the catalyst is present is preferably 5 or more, more preferably 7 or more, still more preferably 10 or more. A solid catalyst also having an effect as a packing for the distillation column can also be used.

As a catalyst used in the step ($\alpha$), various types of catalysts known so far can be used. Specific examples of such catalysts include, but are not particularly limited to, alkali metals and alkaline earth metals, such as lithium, sodium, potassium, rubidium, cesium, magnesium, calcium, strontium and barium;

basic compounds, such as hydrides, hydroxides, alkoxides, aryloxides, and amides of alkali metals and alkaline earth metals;
basic compounds, such as carbonates, bicarbonates, and organic acid salts of alkali metals and alkaline earth metals;
tertiary amines, such as triethylamine, tributylamine, trihexylamine and benzyldiethylamine;
nitrogen-containing heteroaromatic compounds, such as N-alkylpyrrole, N-alkylindole, oxazole, N-alkylimidazole, N-alkylpyrazole, oxadiazole, pyridine, alkylpyridine, quinoline, alkylquinoline, isoquinoline, alkylisoquinoline, acridine, alkylacridine, phenanthroline, alkylphenanthroline, pyrimidine, alkylpyrimidine, pyradine, alkylpyradine, triazine, and alkyltriazine;
cyclic amidines, such as diazabicycloundecene (DBU) and diazabicyclononene (DBN);
thallium compounds, such as thallium oxide, thallium halides, thallium hydroxide, thallium carbonate, thallium nitrate, thallium sulfate, and thallium salts of organic acids;
tin compounds, such as tributylmethoxytin, tributylethoxytin, dibutyldimethoxytin, diethyldiethoxytin, dibutyldiethoxytin, dibutylphenoxytin, diphenylmethoxytin, dibutyltin acetate, tributyltin chloride, and tin 2-ethylhexanoate;
zinc compounds, such as dimethoxyzinc, diethoxyzinc, ethylenedioxyzinc, and dibutoxyzinc;
aluminum compounds, such as aluminum trimethoxide, aluminum triisopropoxide, and aluminum tributoxide;
titanium compounds, such as tetramethoxytitanium, tetraethoxytitanium, tetrabutoxytitanium, dichlorodimethoxytitanium, tetraisopropoxytitanium, titanium acetate, and titanium acetylacetonate;
phosphorus compounds, such as trimethylphosphine, triethylphosphine, tributylphosphine, triphenylphosphine, tributylmethylphosphonium halides, trioctylbutylphosphonium halides, and triphenylmethylphosphonium halides;
zirconium compounds, such as zirconium halides, zirconium acetylacetonate, zirconium alkoxides, and zirconium acetate;
lead and lead-containing compounds, for example, lead oxides, such as PbO, $PbO_2$, and $Pb_3O_4$;
lead sulfides, such as PbS, $Pb_2S_3$, and $PbS_2$;
lead hydroxides, such as $Pb(OH)_2$, $Pb_3O_2(OH)_2$, $Pb_2[PbO_2(OH)_2]$, and $Pb_2O(OH)_2$;
plumbites, such as $Na_3PbO_2$, $K_2PbO_2$, $NaHPbO_3$, and $KHPbO_2$;
plumbates, such as $Na_2PbO_3$, $Na_2H_2PbO_4$, $K_2PbO_3$, $K_2[Pb(OH)_6]$, $K_4PbO_4$, $Ca_2PbO_4$, and $CaPbO_3$;
lead carbonates and basic salts thereof, such as $PbCO_3$, and $2PbCO_3 \cdot Pb(OH)_2$;
alkoxylead compounds and aryloxylead compounds, such as Pb $(OCH_3)_2$, $(CH_3O)Pb(OPh)$, and $Pb(OPh)_2$;
lead salts of organic acids, and carbonates and basic salts thereof, such as $Pb(OCOCH_3)_2$, $Pb(OCOCH_3)_4$, and $Pb(OCOCH_3)_2 \cdot PbO \cdot 3H_2O$;
organolead compounds, such as $Bu_4Pb$, $Ph_4Pb$, $Bu_3PbCl$, $Ph_3PbBr$, $Ph_3Pb$ (or $Ph_6Pb_2$), $Bu_3PbOH$, $Ph_2PbO$, wherein Bu represents a butyl group, and Ph represents a phenyl group;
lead alloys, such as Pb-Na, Pb-Ca, Pb-Ba, Pb-Sn, and Pb-Sb; and
lead minerals, such as galena, and hydrates of these lead compounds.

These compounds each can be used as a homogeneous catalyst when soluble in a reaction starting material, a reaction mixture, a reaction by-product, or the like, and can be used as a solid catalyst when insoluble therein. Further, use of a mixture provided by dissolving these compounds in advance with a reaction starting material, a reaction mixture, a reaction by-product, or the like or reacting these compounds to be dissolved, as a homogeneous catalyst, is also a preferable method.

Further, ion-exchangers, such as anion-exchange resins having tertiary amino groups, ion-exchange resins having amide groups, ion-exchange resins having at least one exchange group of sulfonate, carboxylate, and phosphate groups, and strongly basic solid anion-exchangers having quaternary ammonium groups as exchange groups; solid inorganic compounds, such as silica, silica-alumina, silica-magnesia, aluminosilicate, gallium silicate, various types of zeolites, various types of metal-exchanged zeolites, and ammonium-exchanged zeolites; and the like each are used as a catalyst.

Solid catalysts particularly preferably used are strongly basic solid anion-exchangers having quaternary ammonium groups as exchange groups, and examples thereof include, but are not particularly limited to, strongly basic

anion-exchange resins having quaternary ammonium groups as exchange groups, cellulose type strongly basic anion-exchangers having quaternary ammonium groups as exchange groups, and strongly basic anion-exchangers carried on an inorganic carrier which have quaternary ammonium groups as exchange groups. As strongly basic anion-exchange resins having quaternary ammonium groups as exchange groups, for example, styrene type strongly basic anion-exchange resins and the like are preferably used, but not particularly limited thereto. A styrene type strongly basic anion-exchange resin is a strongly basic anion-exchange resin comprising a copolymer of styrene and divinylbenzene as a base and quaternary ammonium groups (type I or type II) as exchange groups, and schematically represented by the following formulas.

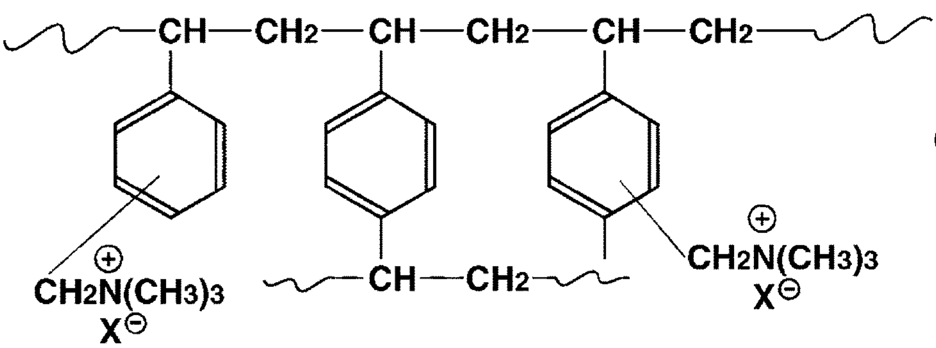

(type I)

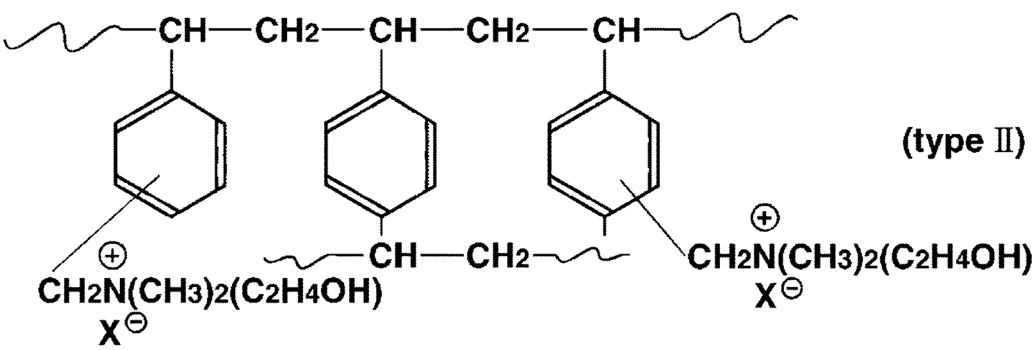

(type II)

In the above formulas, X represents an anion. As X, usually, at least one type of anion selected from $F^-$, $Cl^-$, $Br^-$, $I^-$, $HCO_3^-$, $CO_3^{2-}$, $CH_3CO_2^-$, $HCO_2^-$, $IO_3^-$, $BrO_3^-$, and $ClO_3^-$ is used, and at least one type of anion selected from $Cl^-$, $Br^-$, $HCO_3^-$, and $CO_3^{2-}$ is preferably used. As the structure of the resin base, either a gel type or a macroreticular type (MR type) can be used, but in respect of high resistance to organic solvents, the MR type is particularly preferred.

Examples of cellulose type strongly basic anion-exchangers having quaternary ammonium groups as exchange groups include, but are not particularly limited to, cellulose having exchange groups of $-OCH_2CH_2NR_3X$ provided by trialkylaminoethylation of some or all of the -OH groups of cellulose. Here, R represents an alkyl group. A methyl group, an ethyl group, a propyl group, a butyl group, or the like is usually used, and a methyl group or an ethyl group is preferably used. X represents an anion as mentioned above.

The strongly basic anion-exchanger carried on an inorganic carrier usable in the step ($\alpha$), which has quaternary ammonium groups as exchange groups, means one having quaternary ammonium groups of $-O(CH_2)_nNR_3X$ introduced by modification of some or all of the surface hydroxyl groups -OH on the surface of the inorganic carrier. Here, R and X are as mentioned above. n is usually an integer of 1 to 6, and n is preferably 2. As the inorganic carrier, silica, alumina, silica-alumina, titania, zeolite, or the like can be used, silica, alumina, or silica-alumina is preferably used, and silica is particularly preferably used. As a method for modifying hydroxyl groups on the surface of the inorganic carrier, any method can be used.

As a solid strongly basic anion-exchanger having quaternary ammonium groups as exchange groups, commercially available one can also be used. In such a case, one is ion-exchanged with a desired anion species for a pretreatment in advance and then can be used as a transesterification catalyst.

A solid catalyst comprised of a macroreticular or gel type organic polymer having bonded thereto a heterocyclic group containing at least one nitrogen atom or of an inorganic carrier having bonded thereto a heterocyclic group containing at least one nitrogen atom is preferably used as a transesterification catalyst. Further, a solid catalyst in which some or all of these nitrogen-containing heterocyclic groups are quaternarized is also used as well. Solid catalysts such as ion-exchangers can also serve as a packing in the present embodiment.

The amount of the catalyst to be used in the step ($\alpha$) varies depending on the type of the catalyst to be used.

When the homogeneous catalyst soluble in the reaction liquid under the reaction conditions is fed continuously, the amount thereof used is preferably from 0.0001 to 50% by mass, more preferably from 0.005 to 20% by mass, still more preferably from 0.01 to 10% by mass, based on the total mass of the cyclic carbonate and aliphatic monohydric alcohol as the feed starting material. When the solid catalyst is placed and used in the distillation column, the amount of the catalyst to be used is preferably from 0.01 to 75% by volume, more preferably from 0.05 to 60% by volume, still more preferably from 0.1 to 60% by volume, based on the empty column volume of the distillation column.

When a cyclic carbonate is continuously fed to the continuous multi-stage distillation column A as the reactive distillation column in the step ($\alpha$), the cyclic carbonate is preferably fed to a specific stage. In other words, the cyclic carbonate as a starting material is preferably continuously introduced from one or more introduction ports, provided between the 3rd stage or lower from the top of the continuous multi-stage distillation column and the (n/3)th stage from the top of the continuous multi-stage distillation column, into the continuous multi-stage distillation column. The stages above the cyclic carbonate introduction ports are important in order that high boiling point compounds such as the cyclic carbonate and diol are not contained in the column top component. In this sense, the number of stages above the cyclic carbonate introduction ports is preferably 3 or more, more preferably 4 to 10, still more preferably 5 to 8.

A preferred cyclic carbonate to be used in the step ($\alpha$) is, for example, a halogen-free one produced by a reaction of an alkylene oxide such as ethylene oxide, propylene oxide, or styrene oxide with carbon dioxide. Accordingly, these starting material compounds and a cyclic carbonate containing a small amount of a diol can be used as starting materials for the step ($\alpha$).

In the step ($\alpha$), a dialkyl carbonate and/or a diol as a product may be contained in the feed starting material. The content of the dialkyl carbonate is preferably from 0 to 40% by mass, more preferably from 0 to 30% by mass, still more preferably from 0 to 20% by mass, in terms of % by mass of the dialkyl carbonate in the aliphatic monohydric alcohol/dialkyl carbonate mixture, and the content of the diol is preferably from 0 to 10% by mass, more preferably from 0 to 7% by mass, still more preferably from 0 to 5% by mass, in terms of % by mass in the cyclic carbonate/diol mixture.

When the reaction in the step ($\alpha$) is industrially performed, it is preferred that a substance containing, as the main component, the cyclic carbonate and/or aliphatic monohydric alcohol recovered in the aforementioned steps (I), (II) and/or other steps can be used as the starting material, in addition to a cyclic carbonate and/or an aliphatic monohydric alcohol to be newly introduced into the reaction system. For example, the column top component ($B_T$) to be separated and purified in the step (I), which is usually a mixture with a dialkyl carbonate containing an aliphatic monohydric alcohol as the main component, is preferably reused as a portion of the starting material for the step ($\alpha$). The production method of a dialkyl carbonate of the present embodiment enables this reuse, and this is one of preferred aspects of the production method of a dialkyl carbonate of the present embodiment. Examples of other steps include a step of producing a diaryl carbonate from a dialkyl carbonate and an aromatic monohydroxy compound. In this step, an aliphatic monohydric alcohol is by-produced and recovered. This recovered and by-produced aliphatic monohydric alcohol usually contains a dialkyl carbonate. Further, this recovered and by-produced aliphatic monohydric alcohol may contain an aromatic monohydroxy compound, an alkylarylether, a small amount of an alkylaryl carbonate, a diaryl carbonate, and the like. In the present embodiment, this by-produced aliphatic monohydric alcohol may be used as is as a starting material, or may be used as a starting material after reduction of the amount of contained substances having a boiling point higher than that of the aliphatic monohydric alcohol.

When an aliphatic monohydric alcohol is continuously fed to the continuous multi-stage distillation column A as the reactive distillation column in the step ($\alpha$), the alcohol is preferably fed to a specific stage. For example, the aliphatic monohydric alcohol as a starting material is preferably continuously introduced from one or more introduction ports, provided between the (n/3)th stage or lower from the top of the continuous multi-stage distillation column A and the (2n/3)th stage from the top of the continuous multi-stage distillation column A, into the continuous multi-stage distillation column. When the aliphatic monohydric alcohol to be used as a starting material in the step ($\alpha$) contains a specific amount of a dialkyl carbonate, the introduction port(s) is (are) preferably provided in the aforementioned specific stage(s). More preferred is a case in which the aliphatic monohydric alcohol is continuously introduced from one or more introduction ports provided between the (2n/5)th stage or lower from the top of the continuous multi-stage distillation column and the (3n/5)th stage from the top of the continuous multi-stage distillation column into the continuous multi-stage distillation column.

The starting materials are continuously fed in a liquid form, a gaseous form, or as a gas-liquid mixture to the distillation column. In addition to the feeding of the starting materials to the distillation column as described above, intermittently or continuously feeding starting materials in a gaseous form additionally to the center and/or the lower portion of the distillation column is also a preferred method. A method in which a cyclic carbonate in a liquid form or a gas-liquid mixture state is continuously fed to a stage at a level higher than the stage where the catalyst is present in the distillation column, and the aliphatic monohydric alcohol in a gaseous form and/or a liquid form is continuously fed through one or more introduction ports provided in the above-described stage in the distillation column is also a preferred method. Then, these starting materials are preferably brought into contact with a catalyst in a region of preferably 5 stages or more, more preferably 7 stages or more, still more preferably 10 stages or more of the distillation column.

In the step ($\alpha$), the amount ratio of the aliphatic monohydric alcohol to the cyclic carbonate to be fed to the reactive distillation column may vary depending on the type and amount of the transesterification catalyst and the reaction conditions. Preferably, the aliphatic monohydric alcohol can be fed at a molar ratio in the range of 0.01 to 1,000 with respect to the cyclic carbonate to be fed. The aliphatic monohydric alcohol is preferably fed in an excess amount of 2 times or more by mole in order to increase the conversion of the cyclic carbonate, but use of a too large excess requires a larger apparatus. In such a sense, the molar ratio of the aliphatic monohydric alcohol to the cyclic carbonate is preferably from 2 to 20, still more preferably from 3 to 15, even still more preferably from 5 to 12. When a large amount of unreacted cyclic carbonate remains, the unreacted cyclic carbonate reacts with the diol as the product to by-produce multimers such as a dimer and a trimer. Thus, in the case of industrial implementation, the amount of the unreacted cyclic carbonate remaining is preferably reduced as much as possible.

In the production method of a dialkyl carbonate of the present embodiment, preferably, 2 ton/hr or more of the dialkyl carbonate is continuously produced (step ($\alpha$)) and separated and purified (steps (I) and (II)). The minimum amount of the cyclic carbonate to be continuously fed therefor is preferably 2.2 P ton/hr, more preferably 2.1 P ton/hr, still more preferably 2.0 P ton/hr, based on the amount of the dialkyl carbonate to be produced (P ton/hr). In a still more preferred case, the amount can be made smaller than 1.9 P ton/hr.

The continuous multi-stage distillation column A according to the step ($\alpha$) combines not only conditions from a mere distillation function but also conditions required to allow the reaction stably at a high conversion and also at a high selectivity. Specifically, the column A is preferably a continuous multi-stage distillation column satisfying the following conditions.

A plate column that has a cylindrical trunk portion having a length $L_0$ (cm) and an inner diameter $D_0$ (cm) satisfying the following formulas (1) to (6) and has therein n plates having a plurality of pores, the plate column having a gas withdrawal port having an inner diameter $d_{01}$ (cm) at the column top portion or in the upper portion of the column near to the top, a liquid withdrawal port having an inner diameter of $d_{02}$ (cm) at the column bottom portion or in the lower portion of the column near to the bottom, one or more first introduction ports in the upper portion and/or the middle portion of the column below the gas withdrawal port, and one or more second introduction ports in the middle portion and/or the lower portion of the column above the liquid withdrawal port:

$$2100 \leq L_0 \leq 8000 \quad \text{Formula (1)}$$

$$180 \leq D_0 \leq 2000 \quad \text{Formula (2)}$$

$$4 \leq L_0/D_0 \leq 40 \quad \text{Formula (3)}$$

$$20 \leq n_0 \leq 120 \quad \text{Formula (4)}$$

$$3 \leq D_0/d_{01} \leq 20 \quad \text{Formula (5)}$$

$$5 \leq D_0/d_{02} \leq 30 \quad \text{Formula (6)}.$$

The term "the column top portion or the upper portion of the column near to the top" used in the present embodiment means the portion from the column top portion downward as far as approximately 0.25 $L_0$, and the term "the column bottom portion or the lower portion of the column near to the bottom" means the portion from the column bottom portion upward as far as approximately 0.25 $L_0$. "$L_0$" is as defined above.

Use of a continuous multi-stage distillation column that simultaneously satisfies the formulae (1), (2), (3), (4), (5), and (6) enables a dialkyl carbonate and a diol to be produced from a cyclic carbonate and an aliphatic monohydric alcohol on an industrial scale of preferably 2 ton/hr or more of the dialkyl carbonate and/or preferably 1.3 ton/hr or more of the diol with a high conversion, high selectivity, and high productivity further stably for a long period of time of, for example, 1000 hours or longer, preferably 3000 hours or longer, more preferably 5000 hours or longer.

When $L_0$ (cm) is 2100 or more, the conversion increases, and thus the production amount mentioned above can be achieved. Further, in order to lower the equipment cost while securing the conversion enabling the production amount mentioned above to be attained, $L_0$ is preferably set to 8000 or less. A more preferable range for $L_0$ (cm) is $2300 \leq L_0 \leq 6000$, still more preferably $2500 \leq L_0 \leq 5000$.

When $D_0$ (cm) is 180 or more, the production amount mentioned above can be achieved. Further, in order to lower the equipment cost while achieving the intended production amount, $D_0$ is preferably set to 2000 or less. A more preferable range for $D_0$ (cm) is $200 \leq D_0 \leq 1000$, still more preferably $210 \leq D_0 \leq 800$.

When $L_0/D_0$ is 4 or more and 40 or less, stable operation is facilitated. Particularly when $L_0/D_0$ is 40 or less, an increase in the pressure difference between the top and bottom of the column can be suppressed. Then, prolonged stable operation is facilitated, additionally it is not necessary to raise the temperature in the lower portion of the column, and thus a side reaction tends be suppressed to thereby improve the selectivity. A more preferable range for $L_0/D_0$ is $5 \leq L_0/D_0 \leq 30$, still more preferably $7 \leq L_0/D_0 \leq 20$.

When $n_0$ is 10 or more, the conversion increases, and thus the production mentioned above can be achieved. Further, in order to lower the equipment cost while securing the conversion enabling the intended production amount to be attained, $n_0$ is preferably set to 120 or less. Further, when $n_0$ is 120 or less, an increase in the pressure difference between the top and bottom of the column can be suppressed. Then, prolonged stable operation is facilitated, additionally it is not necessary to raise the temperature in the lower portion of the column, and thus by-reaction tends be suppressed to thereby improve the selectivity. A more preferable range for $n_0$ is $30 \leq n_0 \leq 100$, still more preferably $40 \leq n_0 \leq 90$.

When $D_0/d_{01}$ is 3 or more, not only the equipment cost becomes inexpensive, but also the amount of the gas component discharged out of the system can be suppressed, and thus stable operation is facilitated. When $D_0/d_{01}$ is 20 or less, not only the amount of the gas component to be withdrawn relatively becomes larger and stable operation is facilitated, but also the conversion tends to increase. A more preferable range for $D_0/d_{01}$ is $4 \leq D_0/d_{01} \leq 15$, still more preferably $5 \leq D_0/d_{01} \leq 13$.

When $D_0/d_{02}$ is 5 or more, not only the equipment cost becomes lower, but also the amount of the liquid to be withdrawn relatively becomes smaller, and stable operation is facilitated. When $D_0/d_{02}$ is 30 or less, an abrupt increase in the flow velocity at the liquid withdrawal port or piping can be suppressed, erosion is more unlikely to occur, and thus corrosion of the apparatus can be suppressed. A more preferable range for $D_0/d_{02}$ is $7 \leq D_0/d_{02} \leq 25$, still more preferably $9 \leq D_0/d_{02} \leq 20$.

Further, in the present embodiment, a case in which the $d_{01}$ and the $d_{02}$ satisfy the formula (7) is found to be further preferred:

$$1 \leq d_{01}/d_{02} \leq 5 \quad \text{Formula(7)}.$$

The term "prolonged stable operation" referred to in the present embodiment means that operation can be continued in a steady state based on the operating conditions without flooding, weeping, clogging of piping, or erosion for 1000 hours or longer, preferably 3000 hours or longer, more preferably 5000 hours or longer, and predetermined amounts of the dialkyl carbonate and the diol are produced while the high conversion, high selectivity, and high productivity are maintained.

In the step ($\alpha$), stable production of the dialkyl carbonate and the diol each tends to be enabled for a prolonged period of time with high selectivity and preferably with high productivity for the dialkyl carbonate of 2 ton/hr or more and high productivity for the diol of 1.3 ton/hr or more. The dialkyl carbonate and the diol are more preferably produced in an amount of 3 ton/hr or more and 1.95 ton/hr or more respectively, still more preferably 4 ton/hr or more and 2.6 ton/hr or more respectively. In the step ($\alpha$), when $L_0$, $D_0$, $L_0/D_0$, $n_0$, $D_0/d_{01}$, and $D_0/d_{02}$ of the continuous multi-stage distillation column satisfy $2300 \leq L_0 \leq 6000$, $200 \leq D_0 \leq 1000$, $5 \leq L_0/D_0 \leq 30$, $30 \leq n_0 \leq 100$, $4 \leq D_0/d_{01} \leq 15$, and $7 \leq D_0/d_{02} \leq 25$, respectively, production of 2.5 ton/hr or more, preferably 3 ton/hr or more, still more preferably 3.5 ton/hr or more of the dialkyl carbonate and 1.6 ton/hr or more, preferably 1.95 ton/hr or more, still more preferably 2.2 ton/hr or more of the diol tends to be enabled. Further in the step ($\alpha$), when $L_0$, $D_0$, $L_0/D_0$, $n_0$, $D_0/d_{01}$, and $D_0/d_{02}$ of the continuous multi-stage distillation column satisfy $2500 \leq L_0 \leq 5000$, $210 \leq D_0 \leq 800$, $7 \leq L_0/D_0 \leq 20$, $40 \leq n_0 \leq 90$, $5 \leq D_0/d_{01} \leq 13$, and $9 \leq D_0/d_{02} \leq 20$, respectively, production of 3 ton/hr or more, preferably 3.5 ton/hr or more, still more preferably 4 ton/hr or more of the dialkyl carbonate and 1.95 ton/hr or more, preferably 2.2 ton/hr or more, still more preferably 2.6 ton/hr or more of the diol tends to be enabled.

The selectivity for each of the dialkyl carbonate and the diol referred to in the present embodiment is based on the cyclic carbonate reacted. In the present embodiment, the selectivity is preferably a high selectivity of 95% or more, more preferably a high selectivity of 97% or more, still more preferably a high selectivity of 99% or more. The conversion referred to in the present embodiment usually indicates the conversion of the cyclic carbonate, and in the present embodiment, the conversion of the cyclic carbonate is preferably 95% or more, more preferably 97% or more, still more preferably 99% or more, yet still more preferably 99.5% or more, even still more preferably 99.9% or more.

The continuous multi-stage distillation column A used in the step ($\alpha$) is preferably a plate column-type distillation column having trays as internals. The internal referred to in the present embodiment means an area in the distillation column at which gas and liquid are actually brought into contact with each other. Examples of such trays include, but are not particularly limited to, a bubble-cap tray, a sieve tray, a ripple tray, a ballast tray, a valve tray, a counterflow tray,

a Unifrax tray, a Superfrac tray, a Maxfrac tray, a dual flow tray, a grid plate tray, a turbogrid plate tray, and a Kittel tray. In the step ($\alpha$), a multi-stage distillation column having a portion packed with packings and a tray portion in combination in a part of the plate section can also be used. Examples of packings include, but are not particularly limited to, irregular packings such as a Raschig ring, a Lessing ring, a Pall ring, a Berl saddle, an Intalox saddle, a Dixon packing, a McMahon packing, or Heli-Pak, or regular packings such as Mellapak, Gempak, Techno-pack, Flexipac, a Sulzer packing, a Goodroll packing, or Glitschgrid. The term "numbers of stages n (such as $n_0$, $n_1$, or $n_2$) of the internals" referred to in the present embodiment means the number of trays, and the theoretical number of stages in the case of packings. Accordingly, the number of stages n in the case of multi-stage distillation column having a tray portion and a portion packed with packings in combination is the sum of the number of trays and the theoretical number of stages.

For the reaction between the cyclic carbonate and the aliphatic monohydric alcohol in the step ($\alpha$), either a plate-type continuous multi-stage distillation column in which the internals comprise trays having a predetermined number of stages and/or packings and/or a packed-type continuous multi-stage distillation column may be used, but a plate-type distillation column in which the internals are trays is more preferred. Furthermore, sieve trays each having a sieve portion and a downcomer portion are particularly excellent in terms of the relationship between performance and equipment cost. The sieve trays each have preferably from 100 to 1000 holes/$m^2$ of the area of the sieve portion. A more preferred number of holes is from 120 to 900 holes/$m^2$, still more preferably from 150 to 800 holes/$m^2$ of the area. The cross-sectional area per hole of the sieve trays is preferably from 0.5 to 5 $cm^2$. A more preferred cross-sectional area per hole is from 0.7 to 4 $cm^2$, still more preferably from 0.9 to 3 $cm^2$. Furthermore, particularly preferred is a case in which the sieve tray each have from 100 to 1000 holes/$m^2$ of the area of the sieve portion, and the cross-sectional area per hole is from 0.5 to 5 $cm^2$. The number of holes of the sieve portion may be the same or may be different in all of the sieves.

The "aperture ratio" of each of the sieve trays of the continuous multi-stage distillation column A referred to in the present embodiment means the ratio of the total area of apertures of each tray through which gas and liquid can pass (the total cross-sectional area of the holes) to the area of the tray having the apertures, in each tray constituting the continuous multi-stage distillation column A. For a tray having a downcomer portion, the area of a portion in which bubbling is substantially occurring excluding the portion is taken as the area of the tray.

The aperture ratio of a sieve tray of the continuous multi-stage distillation column A is preferably in the range of 1.5 to 15%. When the aperture ratio is 1.5% or more, not only the apparatus can be downsized with respect to the required production amount and the equipment cost becomes lower, but also the residence time becomes shorter, and thus a by-reaction (e.g., a reaction of a diol as the reaction product with unreacted cyclic carbonate) can be suppressed. When the aperture ratio is 15% or less, the residence time in each tray becomes longer, and thus the number of stages for achieving a high conversion can be reduced. If the above-described $n_0$ is made larger, no inconvenience is caused. In this sense, the preferred range of the aperture ratio is preferably from 1.7 to 8.0%, still more preferably from 1.9 to 6.0%.

The aperture ratios of each of the trays of the continuous multi-stage distillation column A may all be the same or may be different. In the present embodiment, usually, a multi-stage distillation column in which the aperture ratio of the trays in the upper portion is larger than the aperture ratio of the trays in the lower portion is preferably used.

In the step ($\alpha$), a dialkyl carbonate and a diol are continuously produced by continuously feeding a cyclic carbonate and an aliphatic monohydric alcohol as starting materials into a continuous multi-stage distillation column A in which a catalyst is present, performing reaction and distillation simultaneously in the column, continuously withdrawing a low-purity dialkyl carbonate mixture ($A_T$) containing a dialkyl carbonate generated (preferably in a gaseous form) from the upper portion of the column, and continuously withdrawing a high boiling temperature reaction mixture ($A_B$) containing a diol (preferably in a liquid form) from the lower portion of the column.

The reaction time for the transesterification reaction performed in the step ($\alpha$) is considered to equate to the average residence time of the reaction liquid in the continuous multi-stage distillation column A. The reaction time varies depending on the form of the internals in the distillation column A and the number of stages, the feed rate of the starting materials, the type and amount of the catalyst, the reaction conditions, and the like, and is preferably from 0.1 to 20 hours, more preferably from 0.5 to 15 hours, still more preferably from 1 to 10 hours.

The reaction temperature varies depending on the type of the starting material compounds used and the type and amount of the catalyst, and is preferably from 30 to 300°C. It is preferred to increase the reaction temperature so as to increase the reaction rate, but a higher reaction temperature is more likely to cause a side reaction. The reaction temperature is preferably in the range of 40 to 250°C, more preferably 50 to 200°C, still more preferably 60 to 150°C. In the present embodiment, reactive distillation is performed with the temperature of column bottom set to preferably 150°C or less, more preferably 130°C or less, still more preferably 110°C or less, still more preferably 100°C or less. The reaction pressure varies depending on the type and composition of the starting material compounds used, the reaction temperature, and the like. The reaction pressure may be any of a reduced pressure, normal pressure, or an applied pressure, and is preferably from 1 Pa to $2 \times 10^7$ Pa, more preferably from $10^3$ Pa to $10^7$ Pa, still more preferably from $10^4$ to $5 \times 10^6$ Pa.

Examples

Hereinbelow, the present invention will be concretely described by use of Examples and Comparative Examples. The present invention is not intended to be limited in any way to the following examples.

[Purity of dialkyl carbonate]

The purity of a dialkyl carbonate was measured by gas chromatography. The gas chromatography analysis was performed in accordance with GB/T 33107-2016.

[Content of 2-methoxy ethanol and high boiling point compound]

The content of 2-methoxy ethanol and a high boiling point compound, was measured by gas chromatography. The gas chromatography analysis was performed in accordance with GB/T 33107-2016.

[Content of methanol and ethanol]

The content of methanol and ethanol was measured by gas chromatography. The gas chromatography analysis was performed in accordance with GB/T 33107-2016.

[Water content]

The water content was measured by coulometric titration. The coulometric titration analysis was performed in accordance with JIS K2275-3.

[Content of metal component]

The content of a metal component was measured by an ICP method. The analysis by the ICP method was performed in accordance with JIS G1258.

[Example 1]

Step ($\alpha$)

<Continuous multi-stage distillation column A>

The continuous multi-stage distillation column A used was a plate column-type distillation column having trays as internals.

<Reactive distillation>

3.04 ton/hr of ethylene carbonate in a liquid form having a purity of 99.95% by mass was continuously introduced from an introduction port provided at the 55th stage from the bottom into the distillation column A. 4.2 ton/hr of methanol in a gaseous form (containing 8.95% by mass of dimethyl carbonate) and 9.8 ton/hr of methanol in a liquid form (containing 6.66% by mass of dimethyl carbonate) were continuously introduced from introduction ports provided at the 31st stage from the bottom into the distillation column. The molar ratio of the starting material introduced into the distillation column was methanol/ethylene carbonate = 12.7.

The catalyst used was a homogeneous solution provided by adding 4.8 ton of ethylene glycol to 2.5 ton of KOH (48% by weight aqueous solution), heating the solution to approximately 130°C, gradually reducing the pressure, and carrying out heat treatment for approximately 3 hours at approximately 1300 Pa. This catalyst solution was continuously introduced from an introduction port provided at the 54th stage from the bottom into the distillation column (K concentration: 0.1% by mass based on ethylene carbonate fed). Reactive distillation was performed continuously under conditions of a column bottom temperature of 98°C, a column top pressure of approximately $1.118 \times 10^5$ Pa, and a reflux ratio of 0.42.

The low-purity dimethyl carbonate mixture ($A_T$) continuously withdrawn from the column top portion 1 at 14.2 ton/hr contained, 4.13 ton/hr of dimethyl carbonate, 10.08 ton/hr of methanol, and 3.1 kg/h of 2-methoxy ethanol (hereinbelow, also denoted as "2ME"), and carbon dioxide was 2.1 kg/hr (total: 14.2 ton/hr), and the concentration of dimethyl carbonate was about 29% by mass. The liquid continuously withdrawn from the column bottom portion 2 at 2.8 ton/hr contained 2.2 ton/hr of ethylene glycol, 0.6 ton/hr of methanol, and 5 kg/h of unreacted ethylene carbonate.

Step (I)

<Continuous multi-stage distillation column B1>

The material for the continuous multi-stage distillation column B1 was carbon steel, and sieve trays were used as internals both in the recovery section and the concentration section. The contacting surface area with carbon steel in the liquid phase in the continuous multi-stage distillation column B1 was $2.8 \times 10^{-3}$ $m^2 \cdot min/(kg/Hr)$.

<Separation by distillation of low-purity dimethyl carbonate mixture ($A_T$)>

The low-purity dimethyl carbonate mixture ($A_T$) containing 4.13 ton/hr of dimethyl carbonate, 10.08 ton/hr of methanol, 3.1 kg/hr of 2-methoxy ethanol (hereinbelow, also denoted as "2ME"), and 2.1 kg/hr of carbon dioxide (total: 14.2 ton/hr, concentration of dimethyl carbonate: about 29% by mass) was continuously fed from an introduction port to the continuous multi-stage distillation column B1. The continuous multi-stage distillation column B1 was continuously operated at a temperature of column bottom of about 207°C, a pressure of column bottom of about 1.46 MPa, and a reflux ratio of 3.0.

The column top component ($B_T$) continuously withdrawn at 10.59 ton/hr from the column top portion 1 of the continuous multi-stage distillation column B1 contained 10.07 ton/hr of methanol, 0.52 ton/hr of dimethyl carbonate, and 2.1 kg/hr of carbon dioxide. The concentration of methanol in the column top component ($B_T$) was 95.1% by mass.

The column bottom component ($B_B$) continuously withdrawn at 3.62 ton/hr from the column bottom portion 2 of the continuous multi-stage distillation column B1 contained 3.61 ton/hr of dimethyl carbonate, 7.2 kg/hr of methanol, 0.14 kg/hr of 2ME, and 3.6 kg/hr of a high boiling point compound, and the purity of dimethyl carbonate was 99.8% by mass.

In Examples, a compound having a boiling point higher by 100°C or more than that of dimethyl carbonate under a pressure of 760 mmHg was regarded as a high boiling point compound.

In the column bottom component ($B_B$), the content of 2-methoxy ethanol was 39 mass ppm, and the content of the high boiling point compound was 841 mass ppm.

In the continuous multi-stage distillation column B1, the residence time of liquid in column calculated by the following formula (i) was 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

As a continuous multi-stage distillation column B2, a continuous multi-stage distillation column of which the material was stainless steel and which had a side withdrawal port was used. In this distillation column, with respect to the column diameter $D_{21}$ above the side withdrawal port and the column diameter $D_{22}$ below the side withdrawal port, $D_{21}/D_{22}$ was 0.6. The theoretical number of stages of this distillation column was 13.

<Separation by distillation of column bottom component ($B_B$) >

The column bottom component ($B_B$) containing 3.61 ton/hr of dimethyl carbonate, 7.2 kg/hr of methanol, 0.14 kg/hr of 2ME, and 3.6 kg/hr of a high boiling point compound (total: 3.62 ton/hr, purity of dimethyl carbonate: about 99.8% by mass), provided in the step (I), was continuously fed from an introduction port to the continuous multi-stage distillation column B2. The continuous multi-stage distillation column B2 was continuously operated at a temperature of column bottom of about 92°C, a pressure of column bottom of about 3 kPa, and a reflux ratio of 2.0.

The column top component ($B_t$) continuously withdrawn at 176 kg/hr from the column top portion of the continuous multi-stage distillation column B2 contained 169 kg/hr of dimethyl carbonate and 7.1 kg/hr of methanol. The

column bottom component ($B_b$) continuously withdrawn at 23.6 kg/hr from the column bottom portion of the continuous multi-stage distillation column B2 contained 20.0 kg/hr of dimethyl carbonate, 0.1 kg/hr of 2ME, and 3.5 kg/hr of a high boiling point compound.

The side-cut component ($B_s$) continuously withdrawn in a gaseous form at 3.42 ton/hr from the side withdrawal port of the continuous multi-stage distillation column B2 contained 3.42 ton/hr of dimethyl carbonate, 0.06 kg/hr of methanol, 0.04 kg/hr of 2ME, 0.00034 kg/hr of a high boiling point compound, 0.00013 kg/hr of iron, and 0.012 kg/hr of water, and the purity of dimethyl carbonate was 99.991% by mass.

In the continuous operation, the amount of heat required (amount of vapor) per ton of the side-cut component per hour was 247 kW/t. It has been found from the foregoing that dimethyl carbonate having a purity of 99.99% by mass or more can be produced with a small amount of heat consumption, that is, at a small reflux ratio in Example 1.

In the side-cut component ($B_s$), the content of the high boiling point compound was 20 mass ppm or less, the content of 2ME was 30 mass ppm or less, the metal content was 0.6 mass ppm or less, the water content was 20 mass ppm or less, and the methanol content was 20 mass ppm or less.

[Example 2]

Step (I)

<Continuous multi-stage distillation column B1>

For the continuous multi-stage distillation column B1, Unifrax trays were used as internals both in the recovery section and the concentration section.

<Separation by distillation of low-purity dimethyl carbonate mixture ($A_T$)>

The low-purity dimethyl carbonate mixture ($A_T$) containing 3.86 ton/hr of dimethyl carbonate, 4.16 ton/hr of methanol, 1.76 kg/hr of 2ME, and 2.1 kg/hr of carbon dioxide (total: 8.02 ton/hr, concentration of dimethyl carbonate: about 48% by mass) was continuously fed from an introduction port to the continuous multi-stage distillation column B1. The continuous multi-stage distillation column B1 was continuously operated at a temperature of column bottom of about 207°C, a pressure of column bottom of about 1.46 MPa, and a reflux ratio of 2.0.

The column top component ($B_T$) continuously withdrawn at 4.38 ton/hr from the column top portion 1 of the continuous multi-stage distillation column B1 contained 4.14 ton/hr of methanol, 0.24 ton/hr of dimethyl carbonate, and 2.1 kg/hr of carbon dioxide. The concentration of methanol in the column top component ($B_T$) was 94.5% by mass.

The column bottom component ($B_B$) continuously withdrawn at 3.64 ton/hr from the column bottom portion 2 of the continuous multi-stage distillation column B1 contained 3.62 ton/hr of dimethyl carbonate, 24.0 kg/hr of methanol, and 2.06 kg/hr of high boiling point compound, and the purity of dimethyl carbonate was 99.3% by mass.

In the column bottom component ($B_B$), the content of 2-methoxy ethanol was 0 mass ppm, and the content of the high boiling point compound was 566 mass ppm.

In the continuous multi-stage distillation column B1, the residence time of liquid in column calculated by the following formula (i) was 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

The continuous multi-stage distillation column B2 used was a continuous multi-stage distillation column having a side withdrawal port. In this distillation column, with respect to the column diameter $D_{21}$ above the side withdrawal

port and the column diameter $D_{22}$ below the side withdrawal port, $D_{21}/D_{22}$ was 0.6. The theoretical number of stages of this distillation column was 13.

<Separation by distillation of column bottom component ($B_B$) >

The column bottom component ($B_B$) containing 3.62 ton/hr of dimethyl carbonate, 24.0 kg/hr of methanol, and 2.06 kg/hr of a high boiling point compound (total: 3.64 ton/hr, purity of dimethyl carbonate: about 99.3% by mass), provided in the step (I), was continuously fed from an introduction port to the continuous multi-stage distillation column B2. The continuous multi-stage distillation column B2 was continuously operated at a temperature of column bottom of about 92°C, a pressure of column bottom of about 3 kPa, and a reflux ratio of 2.0.

The column top component ($B_t$) continuously withdrawn at 148 kg/hr from the column top portion of the continuous multi-stage distillation column B2 contained 124 kg/hr of dimethyl carbonate and 23.9 kg/hr of methanol. The column bottom component ($B_b$) continuously withdrawn at 24.5 kg/hr from the column bottom portion of the continuous multi-stage distillation column B contained 21.6 kg/hr of dimethyl carbonate and 2.06 kg/hr of a high boiling point compound.

The side-cut component ($B_s$) continuously withdrawn in a gaseous form at 3.47 ton/hr from the side withdrawal port of the continuous multi-stage distillation column B2 contained 3.47 ton/hr of dimethyl carbonate and 0.07 kg/hr of methanol, and the purity of dimethyl carbonate was 99.998% by mass.

In the continuous operation, the amount of heat required (amount of vapor) per ton of the side-cut component per hour was 240 kW/t. It has been found from the foregoing that dimethyl carbonate having a purity of 99.99% by mass or more can be produced with a small amount of heat consumption (at a small reflux ratio) in Example 2.

In the side-cut component ($B_s$), the content of the high boiling point compound was 20 mass ppm or less, the content of 2ME was 30 mass ppm or less, the metal content was 0.6 mass ppm or less, the water content was 20 mass ppm or less, and the methanol content was 20 mass ppm or less.

[Example 3]

Step (I)

<Continuous multi-stage distillation column B1>

For the continuous multi-stage distillation column B1, sieve trays were used as internals both in the recovery section and the concentration section.

<Separation by distillation of low-purity dimethyl carbonate mixture ($A_T$)>

The low-purity dimethyl carbonate mixture ($A_T$) containing 3.86 ton/hr of dimethyl carbonate, 0.48 ton/hr of methanol, 0.91 kg/hr of 2ME, and 2.1 kg/hr of carbon dioxide (total: 4.35 ton/hr, concentration of dimethyl carbonate: about 89% by mass) was continuously fed from an introduction port to the continuous multi-stage distillation column B1. The continuous multi-stage distillation column B1 was continuously operated at a temperature of column bottom of about 207°C, a pressure of column bottom of about 1.46 MPa, and a reflux ratio of 2.0.

The column top component ($B_T$) continuously withdrawn at 0.56 ton/hr from the column top portion 1 of the continuous multi-stage distillation column B1 contained 0.46 ton/hr of methanol, 0.10 ton/hr of dimethyl carbonate, and 2.1 kg/hr of carbon dioxide. The concentration of methanol in the column top component ($B_T$) was 82.1% by mass.

The column bottom component ($B_B$) continuously withdrawn at 3.78 ton/hr from the column bottom portion 2 of the continuous multi-stage distillation column B1 contained 3.76 ton/hr of dimethyl carbonate, 16.5 kg/hr of methanol, and 1.07 kg/hr of high boiling point compound, and the purity of dimethyl carbonate was 99.6% by mass.

In the column bottom component ($B_B$), the content of 2-methoxy ethanol was 0 mass ppm, and the content of the high boiling point compound was 283 mass ppm.

In the continuous multi-stage distillation column B1, the residence time of liquid in column calculated by the following formula (i) was 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

The continuous multi-stage distillation column B2 used was a continuous multi-stage distillation column having a side withdrawal port. In this distillation column, with respect to the column diameter $D_{21}$ above the side withdrawal port and the column diameter $D_{22}$ below the side withdrawal port, $D_{21}/D_{22}$ was 0.6. The theoretical number of stages of this distillation column was 13.

<Separation by distillation of column bottom component ($B_B$) >

The column bottom component ($B_B$) containing 3.76 ton/hr of dimethyl carbonate, 16.5 kg/hr of methanol, and 1.07 kg/hr of a high boiling point compound (total: 3.78 ton/hr, purity of dimethyl carbonate: about 99.6% by mass), provided in the step (I), was continuously fed from an introduction port to the continuous multi-stage distillation column B2. The continuous multi-stage distillation column B2 was continuously operated at a temperature of column bottom of about 92°C, a pressure of column bottom of about 3 kPa, and a reflux ratio of 2.0.

The column top component ($B_t$) continuously withdrawn at 174 kg/hr from the column top portion of the continuous multi-stage distillation column B2 contained 158 kg/hr of dimethyl carbonate and 16.4 kg/hr of methanol. The column bottom component ($B_b$) continuously withdrawn at 24.9 kg/hr from the column bottom portion of the continuous multi-stage distillation column B contained 23.8 kg/hr of dimethyl carbonate and 1.07 kg/hr of a high boiling point compound.

The side-cut component ($B_s$) continuously withdrawn in a gaseous form at 3.58 ton/hr from the side withdrawal port of the continuous multi-stage distillation column B2 contained 3.58 ton/hr of dimethyl carbonate and 0.06 kg/hr of methanol, and the purity of dimethyl carbonate was 99.998% by mass.

In the continuous operation, the amount of heat required (amount of vapor) per ton of the side-cut component per hour was 244 kW/t. It has been found from the foregoing that dimethyl carbonate having a purity of 99.99% by mass or more can be produced with a small amount of heat consumption (at a small reflux ratio) in Example 3.

In the side-cut component ($B_s$), the content of the high boiling point compound was 20 mass ppm or less, the content of 2ME was 30 mass ppm or less, the metal content was 0.6 mass ppm or less, the water content was 20 mass ppm or less, and the methanol content was 20 mass ppm or less.

[Example 4]

Step (I)

<Continuous multi-stage distillation column B1>

The material for the continuous multi-stage distillation column B1 was carbon steel, and sieve trays were used as internals both in the recovery section and the concentration section.

<Separation by distillation of low-purity dimethyl carbonate mixture ($A_T$)>

The low-purity dimethyl carbonate mixture ($A_T$) containing 4.13 ton/hr of dimethyl carbonate, 10.08 ton/hr of methanol, and 2.1 kg/hr of carbon dioxide (total: 14.2 ton/hr, concentration of dimethyl carbonate: about 29% by mass) was continuously fed from an introduction port to the continuous multi-stage distillation column B1. The continuous multi-stage distillation column B1 was continuously operated at a temperature of column bottom of about 207°C, a pressure of column bottom of about 1.46 MPa, and a reflux ratio of 3.0.

The column top component ($B_T$) continuously withdrawn at 10.59 ton/hr from the column top portion 1 of the continuous multi-stage distillation column B1 contained 10.07 ton/hr of methanol, 0.52 ton/hr of dimethyl carbonate, and 2.1 kg/hr of carbon dioxide. The concentration of methanol in the column top component ($B_T$) was 95.1% by mass.

The column bottom component ($B_B$) continuously withdrawn at 3.62 ton/hr from the column bottom portion 2 of the continuous multi-stage distillation column B1 contained 3.61 ton/hr of dimethyl carbonate, 7.2 kg/hr of methanol, and 1.8 kg/hr of a high boiling point compound, and the purity of dimethyl carbonate was 99.8% by mass.

In Examples, a compound having a boiling point higher by 100°C or more than that of dimethyl carbonate under a pressure of 760 mmHg was regarded as a high boiling point compound.

In the column bottom component ($B_B$), the content of 2-methoxy ethanol was 0 mass ppm, and the content of the high boiling point compound was 499 mass ppm.

In the continuous multi-stage distillation column B1, the residence time of liquid in column calculated by the following formula (i) was 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

As a continuous multi-stage distillation column B2, a continuous multi-stage distillation column of which the material was stainless steel and which had a side withdrawal port was used. In this distillation column, with respect to the column diameter $D_{21}$ above the side withdrawal port and the column diameter $D_{22}$ below the side withdrawal port, $D_{21}/D_{22}$ was 0.6. The theoretical number of stages of this distillation column was 13.

<Separation by distillation of column bottom component ($B_B$) >

The column bottom component ($B_B$) containing 3.61 ton/hr of dimethyl carbonate, 7.2 kg/hr of methanol, and 1.8 kg/hr of a high boiling point compound (total: 3.62 ton/hr, purity of dimethyl carbonate: about 99.8% by mass), provided in the step (I), was continuously fed from an introduction port to the continuous multi-stage distillation column B2.

The continuous multi-stage distillation column B2 was continuously operated at a temperature of column bottom of about 92°C, a pressure of column bottom of about 3 kPa, and a reflux ratio of 2.0.

The column top component ($B_t$) continuously withdrawn at 182 kg/hr from the column top portion of the continuous multi-stage distillation column B2 contained 169 kg/hr of dimethyl carbonate and 7.1 kg/hr of methanol. The column bottom component ($B_b$) continuously withdrawn at 23.6 kg/hr from the column bottom portion of the continuous multi-stage distillation column B2 contained 20.0 kg/hr of dimethyl carbonate and 1.8 kg/hr of a high boiling point compound.

The side-cut component ($B_s$) continuously withdrawn in a gaseous form at 3.42 ton/hr from the side withdrawal port of the continuous multi-stage distillation column B2 contained 3.42 ton/hr of dimethyl carbonate and 0.1 kg/hr of methanol, and the purity of dimethyl carbonate was 99.997% by mass.

In the continuous operation, the amount of heat required (amount of vapor) per ton of the side-cut component per hour was 247 kW/t. It has been found from the foregoing that dimethyl carbonate having a purity of 99.99% by mass or more can be produced with a small amount of heat consumption (at a small reflux ratio) in Example 4.

In the side-cut component ($B_s$), the content of the high boiling point compound was 20 mass ppm or less, the content of 2ME was 30 mass ppm or less, the metal content was 0.6 mass ppm or less, the water content was 20 mass ppm or less, and the methanol content was 20 mass ppm or less.

[Example 5]

Step (I)

<Continuous multi-stage distillation column B1>

As the material for the continuous multi-stage distillation column B1, stainless steel was used. Sieve trays were used as internals both in the recovery section and the concentration section. An iron(II) oxide powder was mixed with the feed liquid, and the mixture was fed at 0.83 kg/hr to this distillation column.

<Separation by distillation of low-purity dimethyl carbonate mixture ($A_T$)>

The low-purity dimethyl carbonate mixture ($A_T$) containing 3.86 ton/hr of dimethyl carbonate, 4.16 ton/hr of methanol, 1.76 kg/hr of 2-methoxy ethanol (hereinbelow, also denoted as "2ME"), and 2.1 kg/hr of carbon dioxide (total: 8.02 ton/hr, concentration of dimethyl carbonate: about 48% by mass) was continuously fed from an introduction port to the continuous multi-stage distillation column B1. The continuous multi-stage distillation column B1 was continuously operated at a temperature of column bottom of about 207°C, a pressure of column bottom of about 1.46 MPa, and a reflux ratio of 2.0.

The column top component ($B_T$) continuously withdrawn at 4.38 ton/hr from the column top portion 1 of the continuous multi-stage distillation column B1 contained 4.14 ton/hr of methanol, 0.24 ton/hr of dimethyl carbonate, and 2.1 kg/hr of carbon dioxide. The concentration of methanol in the column top component ($B_T$) was 94.5% by mass.

The column bottom component ($B_B$) continuously withdrawn at 3.64 ton/hr from the column bottom portion 2 of the continuous multi-stage distillation column B1 contained 3.62 ton/hr of dimethyl carbonate, 24.0 kg/hr of methanol, 2.1 kg/hr of a high boiling point compound, and 0.83 kg/hr of iron(II) oxide, and the purity of dimethyl carbonate was 99.26% by mass.

In Examples, a compound having a boiling point higher by 100°C or more than that of dimethyl carbonate under a pressure of 760 mmHg was regarded as the high boiling point compound. In the column bottom component ($B_B$), the content of 2-methoxy ethanol was 0 mass ppm, and the content of the high boiling point compound was 577 mass ppm.

In the continuous multi-stage distillation column B1, the residence time of liquid in column calculated by the following formula (i) was 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

The continuous multi-stage distillation column B2 used was a continuous multi-stage distillation column having a side withdrawal port. In this distillation column, with respect to the column diameter $D_{21}$ above the side withdrawal port and the column diameter $D_{22}$ below the side withdrawal port, $D_{21}/D_{22}$ was 0.6. The theoretical number of stages of this distillation column was 13.

<Separation by distillation of column bottom component ($B_B$) >

The column bottom component ($B_B$) containing 3.62 ton/hr of dimethyl carbonate, 24.0 kg/hr of methanol, 2.1 kg/hr of a high boiling point compound, and 0.83 kg/hr of iron(II) oxide (total: 3.64 ton/hr, purity of dimethyl carbonate: about 99.26% by mass), provided in the step (I), was continuously fed from an introduction port to the continuous multi-stage distillation column B2. The continuous multi-stage distillation column B2 was continuously operated at a temperature of column bottom of about 92°C, a pressure of column bottom of about 3 kPa, and a reflux ratio of 2.0.

The column top component ($B_t$) continuously withdrawn at 148 kg/hr from the column top portion of the continuous multi-stage distillation column B2 contained 124 kg/hr of dimethyl carbonate and 23.9 kg/hr of methanol.

The column bottom component ($B_b$) continuously withdrawn at 24.5 kg/hr from the column bottom portion of the continuous multi-stage distillation column B contained 21.6 kg/hr of dimethyl carbonate, 2.1 kg/hr of a high boiling point compound, and 0.83 kg/hr of iron(II) oxide.

The side-cut component ($B_s$) continuously withdrawn in a gaseous form at 3.47 ton/hr from the side withdrawal port of the continuous multi-stage distillation column B2 contained 3.47 ton/hr of dimethyl carbonate and 0.07 kg/hr of methanol, and the purity of dimethyl carbonate was 99.998% by mass.

In the continuous operation, the amount of heat required (amount of vapor) per ton of the side-cut component per hour was 240 kW/t. It has been found from the foregoing that dimethyl carbonate having a purity of 99.99% by mass or more can be produced with a small amount of heat consumption (at a small reflux ratio) in Example 5.

In the side-cut component ($B_s$), the content of the high boiling point compound was 20 mass ppm or less, the content of 2ME was 30 mass ppm or less, the metal content was 0.6 mass ppm or less, the water content was 20 mass ppm or less, and the methanol content was 20 mass ppm or less.

[Example 6]

Step (I)

<Continuous multi-stage distillation column B1>

As the material for the continuous multi-stage distillation column B1, stainless steel was used. Sieve trays were used as internals both in the recovery section and the concentration section. An iron(II) oxide powder was mixed with the feed liquid, and the mixture was fed at 0.83 kg/hr to this distillation column.

<Separation by distillation of low-purity dimethyl carbonate mixture ($A_T$)>

The low-purity dimethyl carbonate mixture ($A_T$) containing 3.86 ton/hr of dimethyl carbonate, 0.36 ton/hr of methanol, 0.91 kg/hr of 2-methoxy ethanol (hereinbelow, also denoted as "2ME"), and 2.1 kg/hr of carbon dioxide (total: 4.22 ton/hr, concentration of dimethyl carbonate: about 91% by mass) was continuously fed from an introduction port to the continuous multi-stage distillation column B1. The continuous multi-stage distillation column B1 was continuously operated at a temperature of column bottom of about 161°C, a pressure of column bottom of about 0.80 MPa, and a reflux ratio of 10.

The column top component ($B_T$) continuously withdrawn at 0.51 ton/hr from the column top portion 1 of the continuous multi-stage distillation column B1 contained 0.16 ton/hr of methanol, 0.34 ton/hr of dimethyl carbonate, and 2.1 kg/hr of carbon dioxide. The concentration of methanol in the column top component ($B_T$) was 31.4% by mass.

The column bottom component ($B_B$) continuously withdrawn at 3.73 ton/hr from the column bottom portion 2 of the continuous multi-stage distillation column B1 contained 3.71 ton/hr of dimethyl carbonate, 15.4 kg/hr of methanol, 1.07 kg/hr of a high boiling point compound, and 0.83 kg/hr of iron(II) oxide, and the purity of dimethyl carbonate was 99.5% by mass.

In Examples, a compound having a boiling point higher by 100°C or more than that of dimethyl carbonate under a pressure of 760 mmHg was regarded as the high boiling point compound.

In the column bottom component ($B_B$), the content of 2-methoxy ethanol was 0 mass ppm, and the content of the high boiling point compound was 287 mass ppm.

In the continuous multi-stage distillation column B1, the residence time of liquid in column calculated by the following formula (i) was 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

The continuous multi-stage distillation column B2 used was a continuous multi-stage distillation column having a side withdrawal port. In this distillation column, with respect to the column diameter $D_{21}$ above the side withdrawal port and the column diameter $D_{22}$ below the side withdrawal port, $D_{21}/D_{22}$ was 0.6. The theoretical number of stages of this distillation column was 13.

<Separation by distillation of column bottom component ($B_B$) >

The column bottom component ($B_B$) containing 3.71 ton/hr of dimethyl carbonate, 15.4 kg/hr of methanol, 1.07 kg/hr of a high boiling point compound, and 0.83 kg/hr of iron(II) oxide (total: 3.73 ton/hr, purity of dimethyl carbonate: about 99.5% by mass), provided in the step (I), was continuously fed from an introduction port to the continuous multi-stage distillation column B2.

The continuous multi-stage distillation column B2 was continuously operated at a temperature of column bottom of about 92°C, a pressure of column bottom of about 3 kPa, and a reflux ratio of 2.0.

The column top component ($B_t$) continuously withdrawn at 233 kg/hr from the column top portion of the continuous multi-stage distillation column B2 contained 218 kg/hr of dimethyl carbonate and 15.3 kg/hr of methanol. The column bottom component ($B_b$) continuously withdrawn at 23.5 kg/hr from the column bottom portion of the continuous multi-stage distillation column B contained 21.6 kg/hr of dimethyl carbonate, 1.07 kg/hr of a high boiling point compound, and 0.83 kg/hr of iron(II) oxide.

The side-cut component ($B_s$) continuously withdrawn in a gaseous form at 3.47 ton/hr from the side withdrawal port of the continuous multi-stage distillation column B2 contained 3.47 ton/hr of dimethyl carbonate and 0.06 kg/hr of methanol, and the purity of dimethyl carbonate was 99.998% by mass.

In the continuous operation, the amount of heat required (amount of vapor) per ton of the side-cut component per hour was 250 kW/t.

It has been found from the foregoing that dimethyl carbonate having a purity of 99.99% by mass or more can be produced with a small amount of heat consumption (at a small reflux ratio) in Example 6.

In the side-cut component ($B_s$), the content of the high boiling point compound was 20 mass ppm or less, the content of 2ME was 30 mass ppm or less, the metal content was 0.6 mass ppm or less, the water content was 20 mass ppm or less, and the methanol content was 20 mass ppm or less.

[Example 7]

Step (I)

<Continuous multi-stage distillation column B1>

As the material for the continuous multi-stage distillation column B1, stainless steel was used. Sieve trays were used as internals both in the recovery section and the concentration section. An iron(II) oxide powder was mixed with the feed liquid, and the mixture was fed at 0.83 kg/hr to this distillation column.

<Separation by distillation of low-purity dimethyl carbonate mixture ($A_T$)>

The low-purity dimethyl carbonate mixture ($A_T$) containing 3.86 ton/hr of dimethyl carbonate, 0.22 ton/hr of methanol, 0.91 kg/hr of 2-methoxy ethanol (hereinbelow, also denoted as "2ME"), and 2.1 kg/hr of carbon dioxide (total: 4.08 ton/hr, concentration of dimethyl carbonate: about 94.5% by mass) was continuously fed from an introduction port to the continuous multi-stage distillation column B1. The continuous multi-stage distillation column B1 was continuously operated at a temperature of column bottom of about 128°C, a pressure of column bottom of about 0.20 MPa, and a reflux ratio of 11.

The column top component ($B_T$) continuously withdrawn at 0.40 ton/hr from the column top portion 1 of the continuous multi-stage distillation column B1 contained 0.22 ton/hr of methanol, 0.18 ton/hr of dimethyl carbonate, and 2.1 kg/hr of carbon dioxide. The concentration of methanol in the column top component ($B_T$) was 55% by mass. The column bottom component ($B_B$) continuously withdrawn at 3.68 ton/hr from the column bottom portion 2 of the continuous multi-stage distillation column B1 contained 3.68 ton/hr of dimethyl carbonate, 2.61 kg/hr of methanol, 1.07 kg/hr of a high boiling point compound, and 0.83 kg/hr of iron(II) oxide, and the purity of dimethyl carbonate was 99.88% by mass.

In Examples, a compound having a boiling point higher by 100°C or more than that of dimethyl carbonate under

a pressure of 760 mmHg was regarded as the high boiling point compound.

In the column bottom component ($B_B$), the content of 2-methoxy ethanol was 0 mass ppm, and the content of the high boiling point compound was 291 mass ppm.

In the continuous multi-stage distillation column B1, the residence time of liquid in column calculated by the following formula (i) was 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

The continuous multi-stage distillation column B2 used was a continuous multi-stage distillation column having a side withdrawal port. In this distillation column, with respect to the column diameter $D_{21}$ above the side withdrawal port and the column diameter $D_{22}$ below the side withdrawal port, $D_{21}/D_{22}$ was 0.6. The theoretical number of stages of this distillation column was 13.

<Separation by distillation of column bottom component ($B_B$) >

The column bottom component ($B_B$) containing 3.68 ton/hr of dimethyl carbonate, 2.61 kg/hr of methanol, 1.07 kg/hr of a high boiling point compound, and 0.83 kg/hr of iron(II) oxide (total: 3.68 ton/hr, purity of dimethyl carbonate: about 99.88% by mass), provided in the step (I), was continuously fed from an introduction port to the continuous multi-stage distillation column B2.

The continuous multi-stage distillation column B2 was continuously operated at a temperature of column bottom of about 92°C, a pressure of column bottom of about 3 kPa, and a reflux ratio of 2.0.

The column top component ($B_t$) continuously withdrawn at 115 kg/hr from the column top portion of the continuous multi-stage distillation column B2 contained 112 kg/hr of dimethyl carbonate and 2.57 kg/hr of methanol. The column bottom component ($B_t$) continuously withdrawn at 20.2 kg/hr from the column bottom portion of the continuous multi-stage distillation column B contained 19.1 kg/hr of dimethyl carbonate, 1.07 kg/hr of a high boiling point compound, and 0.83 kg/hr of iron(II) oxide. The side-cut component ($B_s$) continuously withdrawn in a gaseous form at 3.51 ton/hr from the side withdrawal port of the continuous multi-stage distillation column B2 contained 3.51 ton/hr of dimethyl carbonate and 0.04 kg/hr of methanol, and the purity of dimethyl carbonate was 99.999% by mass.

In the continuous operation, the amount of heat required (amount of vapor) per ton of the side-cut component per hour was 234 kW/t. It has been found from the foregoing that dimethyl carbonate having a purity of 99.99% by mass or more can be produced with a small amount of heat consumption (at a small reflux ratio) in Example 7.

In the side-cut component ($B_s$), the content of the high boiling point compound was 20 mass ppm or less, the content of 2ME was 30 mass ppm or less, the metal content was 0.6 mass ppm or less, the water content was 20 mass ppm or less, and the methanol content was 20 mass ppm or less.

[Comparative Example 1]

Step (I)

<Continuous multi-stage distillation column B1>

For the continuous multi-stage distillation column B1, sieve trays were used as internals both in the recovery section and the concentration section.

<Separation by distillation of low-purity dialkyl carbonate mixture ($A_T$)>

The low-purity dimethyl carbonate mixture ($A_T$) containing 4.13 ton/hr of dimethyl carbonate, 2.1 kg/hr of methanol, and 0.43 kg/hr of 2-methoxy ethanol (total: 4.13 ton/hr, concentration of dimethyl carbonate: 99.95% by mass) was continuously fed from an introduction port to the continuous multi-stage distillation column B1.

The continuous multi-stage distillation column B1 was continuously operated at a temperature of column bottom of about 109°C, a pressure of column bottom of about 80 kPa, and a reflux ratio of 1.8.

The column top component ($B_T$) continuously withdrawn at 465 kg/hr from the column top portion 1 of the continuous multi-stage distillation column B1 contained 2.0 kg/hr of methanol and 463 kg/hr of dimethyl carbonate. The column bottom component ($B_B$) continuously withdrawn at 3.61 ton/hr from the column bottom portion 2 of the continuous multi-stage distillation column B1 contained 3.61 ton/hr of dimethyl carbonate, 0.72 kg/hr of methanol, and 0.43 kg/hr of 2-methoxy ethanol, and the purity of dimethyl carbonate was 99.97% by mass. In the column bottom component ($B_B$), the content of 2-methoxy ethanol was 119 mass ppm, and the content of the high boiling point compound was 0 mass ppm.

In the continuous multi-stage distillation column B1, the residence time of liquid in column calculated by the following formula (i) was about 4 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

The continuous multi-stage distillation column B2 used was a continuous multi-stage distillation column having a side withdrawal port. In this distillation column, the ratio of the column diameter $D_{21}$ above the side withdrawal port to the column diameter $D_{22}$ below the side withdrawal port, $D_{21}/D_{22}$ was 0.83, and the theoretical number of stages was 13.

<Separation by distillation of column bottom component ($B_B$) >

The column bottom component ($B_B$) containing 3.61 ton/hr of dimethyl carbonate, 0.72 kg/hr of methanol, and 0.43 kg/hr of 2-methoxy ethanol (total: 3.61 ton/hr, purity of dimethyl carbonate: 99.97% by mass), provided in the step (I), was continuously fed from an introduction port to the continuous multi-stage distillation column B2. The continuous multi-stage distillation column B2 was continuously operated at a temperature of column bottom of about 92°C, a pressure of column bottom of about 3 kPa, and a reflux ratio of 20.

The column top component ($B_t$) continuously withdrawn at 200 kg/hr from the column top portion of the continuous multi-stage distillation column B2 contained 199 kg/hr of dimethyl carbonate and 0.72 kg/hr of methanol. The column bottom component ($B_b$) continuously withdrawn at 11.1 kg/hr from the column bottom portion of the continuous multi-stage distillation column B2 contained 10.8 kg/hr of dimethyl carbonate and 0.33 kg/hr of 2-methoxy ethanol. The side-cut component ($B_s$) continuously withdrawn in a gaseous form at 3.4 ton/hr from the side withdrawal port of the continuous multi-stage distillation column B2 contained 3.4 ton/hr of dimethyl carbonate and 0.1 kg/hr of 2-methoxy ethanol, and the purity of dimethyl carbonate was 99.997% by mass. In the continuous operation, the amount of heat required (amount of vapor) per ton of the side-cut component per hour was 465 kW/t. From the foregoing, in Comparative Example 1, production of dimethyl carbonate having a purity of 99.99% by mass or more was enabled, but a large amount of heat consumption (a large reflux ratio) was required.

In the side-cut component ($B_s$), the metal content was 0.6 mass ppm or less, the water content was 20 mass ppm or less, and the methanol content was 1 mass ppm or less.

[Comparative Example 2]

Step (I)

<Continuous multi-stage distillation column B1>

For the continuous multi-stage distillation column B1, sieve trays were used as internals both in the recovery section and the concentration section.

<Separation by distillation of low-purity dimethyl carbonate mixture ($A_T$)>

The low-purity dimethyl carbonate mixture ($A_T$) containing 4.13 ton/hr of dimethyl carbonate, 2.1 kg/hr of methanol, and 0.43 kg/hr of 2-methoxy ethanol (total: 4.13 ton/hr, concentration of dimethyl carbonate: 99.95% by mass) was continuously fed from an introduction port to the continuous multi-stage distillation column B1. The continuous multi-stage distillation column B1 was continuously operated at a temperature of column bottom of about 109°C, a pressure of column bottom of about 80 kPa, and a reflux ratio of 1.8.

The column top component ($B_T$) continuously withdrawn at 465 kg/hr from the column top portion 1 of the continuous multi-stage distillation column B1 contained 2.0 kg/hr of methanol and 463 kg/hr of dimethyl carbonate. The column bottom component ($B_B$) continuously withdrawn at 3.61 ton/hr from the column bottom portion 2 of the continuous multi-stage distillation column B1 contained 3.61 ton/hr of dimethyl carbonate, 0.72 kg/hr of methanol, and 0.43 kg/hr of 2-methoxy ethanol, and the purity of dimethyl carbonate was 99.97% by mass. In the column bottom component ($B_B$), the content of 2-methoxy ethanol was 119 mass ppm, and the content of the high boiling point compound was 0 mass ppm.

In the continuous multi-stage distillation column B1, the residence time of liquid in column calculated by the following formula (i) was about 10 minutes.

Residence time of liquid in column (min) = BTM volume (volume of liquid residing in the column BTM during operation (kg))/BTM withdrawal flow rate (flow rate for withdrawal of column bottom component (kg/min))...(i)

Step (II)

<Continuous multi-stage distillation column B2>

The continuous multi-stage distillation column B2 used was a continuous multi-stage distillation column having a side withdrawal port. In this distillation column, the ratio of the column diameter $D_{21}$ above the side withdrawal port to the column diameter $D_{22}$ below the side withdrawal port, $D_{21}/D_{22}$ was 0.42, and the theoretical number of stages was 17.

<Separation by distillation of column bottom component ($B_B$) >

The column bottom component ($B_B$) containing 3.61 ton/hr of dimethyl carbonate, 0.72 kg/hr of methanol, and 0.43 kg/hr of 2-methoxy ethanol (total: 3.61 ton/hr, purity of dimethyl carbonate: 99.97% by mass), provided in the step (I), was continuously fed from an introduction port to the continuous multi-stage distillation column B2. The continuous multi-stage distillation column B2 was continuously operated at a temperature of column bottom of about 92°C, a pressure of column bottom of about 3 kPa, and a reflux ratio of 20.

The column top component ($B_t$) continuously withdrawn at 190 kg/hr from the column top portion of the continuous multi-stage distillation column B2 contained 189 kg/hr of dimethyl carbonate and 0.47 kg/hr of methanol. The column bottom component ($B_b$) continuously withdrawn at 34.2 kg/hr from the column bottom portion of the continuous multi-stage distillation column B2 contained 34.0 kg/hr of dimethyl carbonate and 0.18 kg/hr of 2-methoxy ethanol. The side-cut component ($B_s$) continuously withdrawn in a gaseous form at 3.4 ton/hr from the side withdrawal port of the continuous multi-stage distillation column B2 contained 3.4 ton/hr of dimethyl carbonate, 0.21 kg/hr of methanol, and 0.25 kg/hr of 2-methoxy ethanol, and the purity of dimethyl carbonate was 99.98% by mass. In the continuous operation,

the amount of heat required (amount of vapor) per ton of the side-cut component per hour was 257 kW/t. From the foregoing, in Comparative Example 2, dimethyl carbonate having a purity of 99.99% by mass or more could not be produced.

In the side-cut component ($B_s$), the metal content was 1 mass ppm or less, the water content was 30 mass ppm or less, and the methanol content was 62 mass ppm.

During continuous operation over a long period, in the side-cut component ($B_s$), for any of these, the metal content was 0.6 mass ppm or less, the water content was 20 mass ppm or less, and the methanol content was 20 mass ppm or more.

[Example 8]

Dimethyl carbonate was produced under the same production conditions as in Example 5 except the feed rate of iron(II) oxide was changed as shown in Table 1. Measurement results such as the amount of impurities are shown in Table 1.

[Table 1-1]

| Table 1 (1/2) | | | | | | | |
|---|---|---|---|---|---|---|---|
| | | Example 1 | Example 2 | Example 3 | Example 4 | Example 5 | |
| B1 | Material | Carbon steel | Carbon steel | Carbon steel | Carbon steel | Stainless steel | |
| | Internal | Sieve tray | Unifrax tray | Sieve tray | Sieve tray | Sieve tray | |
| | Iron(II) oxide feed rate | kg/H | - | - | - | - | 0.83 |
| $A_T$ | Flow rate | kg/H | 14210 | 8020 | 4350 | 14210 | 8020 |
| | DMC flow rate | kg/H | 4130 | 3860 | 3860 | 4130 | 3860 |
| | DMC concentration | % by mass | 29 | 48 | 89 | 29 | 48 |
| | ME Flow rate | kg/H | 10080 | 4160 | 480 | 10080 | 4160 |
| | 2ME Flow rate | kg/H | 3.1 | 1.76 | 0.91 | 1.5 | 1.76 |
| | Carbon dioxide flow rate | kg/H | 2.1 | 2.1 | 2.1 | 2.1 | 2.1 |
| | Temperature | °C | 125 | 125 | 125 | 125 | 125 |
| $B_T$ | Flow rate | kg/H | 10587 | 4380 | 560 | 10590 | 4380 |
| | ME Flow rate | kg/H | 10070 | 4140 | 460 | 10070 | 4140 |
| | ME concentration | % by mass | 95.1 | 94.5 | 82.1 | 95.1 | 94.5 |
| | DMC flow rate | kg/H | 520 | 240 | 100 | 520 | 240 |
| | Carbon dioxide flow rate | kg/H | 2.1 | 2.1 | 2.1 | 2.1 | 2.1 |

(continued)

| Table 1 (1/2) | | | | | | | |
|---|---|---|---|---|---|---|---|
| | | Example 1 | Example 2 | Example 3 | Example 4 | Example 5 | |
| $B_B$ | Flow rate | kg/H | 3620 | 3640 | 3780 | 3620 | 3640 |
| | DMC flow rate | kg/H | 3610 | 3620 | 3760 | 3610 | 3620 |
| | DMC concentration | % by mass | 99.8 | 99.3 | 99.6 | 99.8 | 99.26 |
| | ME Flow rate | kg/H | 7.2 | 24 | 16.5 | 7.2 | 24 |
| | 2ME Flow rate | kg/H | 0.14 | 0 | 0 | 0 | 0 |
| | 2ME content | Mass ppm | 39 | 0 | 0 | 0 | 0 |
| | High boiling point compound | kg/H | 3.6 | 2.06 | 1.07 | 1.8 | 2.1 |
| | High boiling point compound content | Mass ppm | 994 | 566 | 283 | 499 | 557 |
| | Iron(II) oxide flow rate | kg/H | - | - | - | - | 0.83 |
| Temperature of column bottom | | °C | 207 | 207 | 207 | 207 | 207 |
| Pressure of column bottom | | Mpa | 1.46 | 1.46 | 1.46 | 1.46 | 1.46 |
| Reflux ratio | | - | 3.0 | 2.0 | 2.0 | 3.0 | 2.0 |
| Residence time in B1 column | | Minutes | 10 | 10 | 10 | 10 | 10 |
| Contacting surface area with iron compound | | $\times 10^{-3}$ $m^2 \cdot min/(kg/H)$ | 2.8 | 4.2 | 8.8 | 2.8 | 545 |
| B2 | Material | | Stainless steel | Stainless steel | Stainless steel | Stainless steel | Stainless steel |
| Bt | Flow rate | kg/H | 176.1 | 147.9 | 174.4 | 176 | 148 |
| | DMC flow rate | kg/H | 169 | 124 | 158 | 169 | 124 |
| | ME Flow rate | kg/H | 7.1 | 23.9 | 16.4 | 7.1 | 23.9 |
| Bb | Flow rate | kg/H | 23.6 | 24.5 | 24.9 | 21.8 | 24.5 |
| | DMC flow rate | kg/H | 20 | 21.6 | 23.8 | 20 | 21.6 |
| | 2ME Flow rate | kg/H | 0.1 | 0 | 0 | 0 | 0 |
| | High boiling point compound | kg/H | 3.5 | 2.1 | 1.0 | 1.8 | 2.1 |
| | Iron(II) oxide flow rate | kg/H | - | - | - | - | 0.83 |

(continued)

| Table 1 (1/2) | | | | | | | |
|---|---|---|---|---|---|---|---|
| | | Example 1 | Example 2 | Example 3 | Example 4 | Example 5 | |
| Bs | Flow rate | kg/H | 3420 | 3470 | 3580 | 3420 | 3470 |
| | DMC flow rate | kg/H | 3420 | 3470 | 3580 | 3420 | 3470 |
| | DMC purity | % by mass | 99.991 | 99.998 | 99.998 | 99.997 | 99.998 |
| | ME Flow rate | kg/H | 0.06 | 0.07 | 0.06 | 0.1 | 0.07 |
| | 2ME Flow rate | kg/H | 0.04 | 0 | 0 | 0 | 0 |
| | High boiling point compound content | Mass ppm | 20 or less | 20 or less | 20 or less | 20 or less | 20 or less |
| | 2ME content | Mass ppm | 30 or less | 30 or less | 30 or less | 30 or less | 30 or less |
| | Metal content | Mass ppm | 0.6 or less | 0.6 or less | 0.6 or less | 0.6 or less | 0.6 or less |
| | Water content | Mass ppm | 20 or less | 20 or less | 20 or less | 20 or less | 20 or less |
| | ME content | Mass ppm | 20 or less | 20 or less | 20 or less | 20 or less | 20 or less |
| | Amount of heat required | kW/t | 247 | 240 | 244 | 247 | 240 |

[Table 1-2]

| Table 1 (2/2) | | | | | | | |
|---|---|---|---|---|---|---|---|
| | | | Example 6 | Example 7 | Example 8 | Comparative Example 1 | Comparative Example 2 |
| B1 | Material | | Stainless steel | Stainless steel | Stainless steel | Stainless steel | Stainless steel |
| | Internal | | Sieve tray | Sieve tray | Sieve tray | Sieve tray | Sieve tray |
| | Iron(II) oxide feed rate | kg/H | 0.83 | 0.83 | 0.02 | - | - |
| $A_T$ | Flow rate | kg/H | 4220 | 4080 | 8027 | 4133 | 4133 |
| | DMC flow rate | kg/H | 3860 | 3860 | 3860 | 4130 | 4130 |
| | DMC concentration | % by mass | 91 | 95 | 48 | 99.95 | 99.95 |
| | ME Flow rate | kg/H | 360 | 220 | 4164 | 2.72 | 2.72 |
| | 2ME Flow rate | kg/H | 0.91 | 0.91 | 0.91 | 0.43 | 0.43 |
| | Carbon dioxide flow rate | kg/H | 2.1 | 2.1 | 2.1 | 0 | 0 |
| | Temperature | °C | 125 | 125 | 125 | 125 | 125 |
| $B_T$ | Flow rate | kg/H | 310 | 400 | 4382 | 522 | 522.0 |
| | ME Flow rate | kg/H | 160 | 220 | 4140 | 2 | 2.0 |
| | ME concentration | % by mass | 48.4 | 55 | 94.5 | 0.38 | 0.38 |
| | DMC flow rate | kg/H | 150 | 180 | 240 | 520 | 520 |
| | Carbon dioxide flow rate | kg/H | 2.1 | 2.1 | 2.1 | 0 | 0 |

(continued)

| Table 1 (2/2) | | | | | | | |
|---|---|---|---|---|---|---|---|
| | | | Example 6 | Example 7 | Example 8 | Comparative Example 1 | Comparative Example 2 |
| $B_B$ | Flow rate | kg/H | 3730 | 3680 | 3645 | 3611 | 3611 |
| | DMC flow rate | kg/H | 3710 | 3680 | 3620 | 3610 | 3610 |
| | DMC concentration | % by mass | 99.5 | 99.88 | 99.26 | 99.97 | 99.97 |
| | ME Flow rate | kg/H | 15.4 | 261 | 24 | 0.72 | 0.72 |
| | 2ME Flow rate | kg/H | 0 | 0 | 0 | 0.43 | 0.43 |
| | 2ME content | Mass ppm | 0 | 0 | 0 | 119 | 119 |
| | High boiling point compound | kg/H | 1.07 | 1.07 | 1.07 | 0 | 0 |
| | High boiling point compound content | Mass ppm | 287 | 291 | 294 | 0 | 0 |
| | Iron(II) oxide flow rate | kg/H | 0.83 | 0.83 | 0.02 | - | - |
| Temperature of column bottom | | °C | 161 | 128 | 207 | 109 | 109 |
| Pressure of column bottom | | Mpa | 0.80 | 0.20 | 1.46 | 0.08 | 0.08 |
| Reflux ratio | | - | 10.0 | 11.0 | 2.0 | 1.8 | 1.8 |
| Residence time in B1 column | | Minutes | 10 | 10 | 8 | 4 | 10 |
| Contacting surface area with iron compound | | $\times 10^{-3}$ $m^2 \cdot min/(kg/H)$ | 1035 | 1071 | 10.5 | - | - |
| B2 | Material | | Stainless steel | Stainless steel | Stainless steel | Stainless steel | Stainless steel |
| Bt | Flow rate | kg/H | 233 | 115 | 148 | 200 | 190 |
| | DMC flow rate | kg/H | 218 | 112 | 124 | 199 | 189 |
| | ME Flow rate | kg/H | 15.3 | 2.57 | 23.9 | 0.72 | 0.47 |
| Bb | Flow rate | kg/H | 23.5 | 20.2 | 24.5 | 11.0 | 21.2 |
| | DMC flow rate | kg/H | 21.6 | 19.1 | 21.6 | 10.8 | 21 |
| | 2ME Flow rate | kg/H | 0 | 0 | 0 | 0.33 | 0.18 |
| | High boiling point compound | kg/H | 1.07 | 1.07 | 1.07 | 0 | 0.0 |
| | Iron(II) oxide flow rate | kg/H | 0.83 | 0.83 | 0.02 | - | - |

(continued)

| Table 1 (2/2) | | | | | | | |
|---|---|---|---|---|---|---|---|
| | | | Example 6 | Example 7 | Example 8 | Comparative Example 1 | Comparative Example 2 |
| Bs | Flow rate | kg/H | 3470 | 3550 | 3474 | 3400 | 3400.5 |
| | DMC flow rate | kg/H | 3470 | 3550 | 3474 | 3400 | 3400 |
| | DMC purity | % by mass | 99.998 | 99.999 | 99.998 | 99.997 | 99.98 |
| | ME Flow rate | kg/H | 0.06 | 0.04 | 0.07 | 0 | 0.21 |
| | 2ME Flow rate | kg/H | 0 | 0 | 0 | 0.1 | 0.25 |
| | High boiling point compound content | Mass ppm | 20 or less | 20 or less | 20 or less | 0 | 0 |
| | 2ME content | Mass ppm | 30 or less | 30 or less | 30 or less | 30 or less | 74 |
| | Metal content | Mass ppm | 0.6 or less | 0.6 or less | 0.6 or less | 0.6 or less | 0.6 or less |
| | Water content | Mass ppm | 20 or less | 20 or less | 20 or less | 20 or less | 30 or less |
| | ME content | Mass ppm | 20 or less | 20 or less | 20 or less | 1 or less | 20 or more |
| | Amount of heat required | kW/t | 240 | 234 | 240 | 465 | 257 |

[Example 9]

Dimethyl carbonate was produced under the same production conditions as in Example 1 except that the apparatus shown in Figure 2 was used, the low-purity dialkyl carbonate mixture ($A_T$) to be introduced to the continuous multi-stage distillation column B1 was heated with a heater (not shown), and the temperature and the time from the completion of heating to the introduction into the continuous multi-stage distillation column B1 (feed time) shown in Table 2 were used. Measurement results such as the amount of impurities are shown in Table 2.

The apparatus shown in Figure 2 has a distillation column of various dimensions shown in Figure 3: D1 = 1.6 m, H1 = 26 m, D2 = 2.2 m, H2 = 10 m, D3 = 0.9 m, and H3 = 2.5 m, as the continuous multi-stage distillation column B1, has a distillation column of various dimensions shown in Figure 3: D1 = 0.85 m, H1 = 28 m, D2 = 1.3 m, and H2 = 12 m, as the continuous multi-stage distillation column B2, and has a reflux drum D in the rear stage of a column top condenser 11 of the continuous multi-stage distillation column B1. In each of the continuous multi-stage distillation column B1 and the continuous multi-stage distillation column B2, H1 is a column upper stage straight body portion, H2 is a column bottom lower stage straight body portion, and a portion connecting therebetween is a taper portion. In the continuous multi-stage distillation column B2, a side withdrawal port (side-cut port) is formed in the taper portion.

[Examples 10 and 11, Comparative Examples 3 to 5]

The procedure was performed in the same manner as in Example 9 except that the heating temperature for the low-purity dimethyl carbonate mixture ($A_T$) and the residence time until introduction from the heater were changed as shown in Table 2. Measurement results such as the amount of impurities are shown in Table 2.

[Comparative Example 6]

The procedure was performed in the same manner as in Example 9 except for changing to the side-cut component (Bs2) of the continuous multi-stage distillation column B2 containing the dialkyl carbonate as the main component withdrawn from the withdrawal port provided in the upper stage straight body portion of the continuous multi-stage distillation column B2. The production conditions and measurement results are shown in Table 2.

[Table 2]

Table 2

| | | | Example 9 | Example 10 | Example 11 | Comparative Example 3 | Comparative Example 4 | Comparative Example 5 | Comparative Example 6 |
|---|---|---|---|---|---|---|---|---|---|
| $A_T$ | Flow rate | kg/H | 14210 | 14210 | 14210 | 14210 | 14210 | 14210 | 14210 |
| | DMC flow rate | kg/H | 4130 | 4130 | 4130 | 4130 | 4130 | 4130 | 4130 |
| | DMC concentration | % by mass | 29 | 29 | 29 | 29 | 29 | 29 | 29 |
| | Other impurities | Mass ppm | 200 | 200 | 200 | 200 | 200 | 200 | 200 |
| | Temperature | °C | 125 | 100 | 150 | 80 | 170 | 125 | 125 |
| | Feed time | Minutes | 3 | 3 | 3 | 3 | 3 | 5.5 | 3 |
| $B_T$ | Flow rate | kg/H | 10587 | 10587 | 10587 | 10587 | 10587 | 10587 | 10587 |
| | ME concentration | % by mass | 95.1 | 95.1 | 95.1 | 95.1 | 95.1 | 95.1 | 95.1 |
| | Temperature | °C | 125 | 125 | 125 | 125 | 125 | 125 | 125 |
| $B_B$ | Flow rate | kg/H | 3620 | 3620 | 3620 | 3620 | 3620 | 3620 | 3620 |
| | DMC concentration | % by mass | 99.8 | 99.8 | 99.8 | 99.8 | 99.8 | 99.8 | 99.8 |
| | Impurities | Mass ppm | 1471 | 1505 | 1493 | 1560 | 1593 | 1549 | 1471 |
| B1v | Flow rate | kg/H | 3 | 3 | 3 | 3 | 3 | 3 | 3 |
| Bt | Flow rate | kg/H | 176.1 | 176.1 | 176.1 | 176.1 | 176.1 | 176.1 | 176.1 |
| | DMC concentration | % by mass | 96 | 96 | 96 | 96 | 96 | 96 | 96 |
| Bb | Flow rate | kg/H | 23.6 | 24.1 | 23.9 | 25.0 | 25.5 | 24.8 | 23.6 |
| | Impurities | % by mass | 22.6 | 22.6 | 22.6 | 22.6 | 22.6 | 22.6 | 22.6 |

(continued)

| | | | | | | | | | |
|---|---|---|---|---|---|---|---|---|---|
| | Feed time | Minutes | 3 | 3 | 3 | 3 | 3 | 5.5 | 3 |
| Bs | Flow rate | kg/H | 3420.3 | 3419.8 | 3420.0 | 3418.9 | 3418.4 | 3419.0 | 3420.3 |
| | DMC purity | % by mass | 99.991 | 99.991 | 99.991 | 99.991 | 99.991 | 99.991 | 99.99 |
| | Metal content | Mass ppm | 0.6 or less | 1 or less | 1 or less | 1 or less | 1 or less | 1 or less | 1.2 |
| | Water content | Mass ppm | 20 or less | 30 or less | 30 or less | 30 or less | 30 or less | 30 or less | 30 or less |
| | ME content | Mass ppm | 20 or less | 20 or less | 20 or less | 20 or less | 20 or less | 20 or less | 30 |
| Annual loss | | ton/yr | Reference | 4.1 | 2.5 | 11.1 | 15.3 | 9.7 | - |

[Examples 12 and 13, Comparative Examples 7 and 8]

The procedure was performed in the same manner as in Example 9 except that the reflux drum temperature of the continuous multi-stage distillation column B1 was changed as described in Table 3. The production conditions and measurement results are shown in Table 3.

[Table 3]

Table 3

| | | | Example 12 | Example 13 | Comparative Example 7 | Comparative Example 8 |
|---|---|---|---|---|---|---|
| $A_T$ | Flow rate | kg/H | 14210 | 14210 | 14210 | 14210 |
| | DMC flow rate | kg/H | 4130 | 4130 | 4130 | 4130 |
| | DMC concentration | % by mass | 29 | 29 | 29 | 29 |
| | Other impurities | Mass ppm | 200 | 200 | 200 | 200 |
| | Temperature | °C | 125 | 125 | 125 | 125 |
| | Feed time | Minutes | 3 | 3 | 3 | 3 |
| $B_T$ | Flow rate | kg/H | 10587.3 | 10586.7 | 10587.7 | 10586.0 |
| | ME concentration | % by mass | 95.1 | 95.1 | 95.1 | 95.1 |
| | Temperature | °C | 100 | 150 | 80 | 170 |
| $B_B$ | Flow rate | kg/H | 3620 | 3620 | 3620 | 3620 |
| | DMC concentration | % by mass | 99.8 | 99.8 | 99.8 | 99.8 |
| | Impurities | Mass ppm | 1493 | 1460 | 1537 | 1471 |
| B1v | Flow rate | kg/H | 2.7 | 3.3 | 2.3 | 4 |
| Bt | Flow rate | kg/H | 176.1 | 176.1 | 176.1 | 176.1 |
| | DMC concentration | % by mass | 96 | 96 | 96 | 96 |
| Bb | Flow rate | kg/H | 23.9 | 23.4 | 24.6 | 23.6 |
| | Impurities | % by mass | 22.6 | 22.6 | 22.6 | 22.6 |
| Bs | Flow rate | kg/H | 3420.0 | 3420.5 | 3419.3 | 3420.3 |
| | DMC purity | % by mass | 99.991 | 99.991 | 99.991 | 99.991 |
| | Metal content | Mass ppm | 1 or less | 1 or less | 1 or less | 1 or less |
| | Water content | Mass ppm | 30 or less | 30 or less | 30 or less | 30 or less |
| | ME content | Mass ppm | 20 or less | 20 or less | 20 or less | 20 or less |
| Annual loss | | ton/yr | 0.1 | 0.7 | 2.6 | 7.7 |

Industrial Applicability

According to the production method of the present invention, it is possible to provide a production method of

a dialkyl carbonate having a high purity of such a level as to enable the dialkyl carbonate to be used for lithium ion battery electrolyte solutions (a purity of 99.99% by mass or more), for example, with a small amount of heat consumption (at a small reflux ratio).

Reference Signs List

B1: first continuous multi-stage distillation column, B2: second continuous multi-stage distillation column, C: industrial dimethyl carbonate tank, D: reflux drum, 11: column top condenser of the continuous multi-stage distillation column B1, 12: reboiler of the continuous multi-stage distillation column B1, 21: column top condenser of the continuous multi-stage distillation column B2, 22: reboiler of the continuous multi-stage distillation column B2, $A_T$: low-purity dialkyl carbonate mixture as a feed to continuous multi-stage distillation column B1, $B_T$: column top component of the continuous multi-stage distillation column B1 containing an aliphatic monohydric alcohol as the main component, $B_B$: column bottom component of the continuous multi-stage distillation column B1 containing a dialkyl carbonate as the main component, $B_t$: column top component of the continuous multi-stage distillation column B2 as a low boiling point component, $B_s$: side-cut component of the continuous multi-stage distillation column B2 containing a dialkyl carbonate as the main component, Bs2: side-cut component of the continuous multi-stage distillation column B2 containing a dialkyl carbonate as the main component from withdrawal port in Comparative Example, $B_b$: column bottom component of the continuous multi-stage distillation column B2 as a high boiling point component, D1: column inner diameter of the upper stage straight body portion, H1: column length of the upper stage straight body portion, D2: column inner diameter of lower stage straight body portion, H2: column length of the lower stage straight body portion

## Claims

1. A production method of a dialkyl carbonate, comprising:

   (I) a first separation and purification step (I) of continuously feeding a low-purity dialkyl carbonate mixture ($A_T$) containing a dialkyl carbonate and an aliphatic monohydric alcohol to a continuous multi-stage distillation column B1, continuously withdrawing a column top component ($B_T$) containing the aliphatic monohydric alcohol as the main component from the upper portion of the column, and continuously withdrawing a column bottom component ($B_B$) containing the dialkyl carbonate as the main component from the lower portion of the column, and
   (II) a second separation and purification step (II) of continuously feeding the column bottom component ($B_B$) containing the dialkyl carbonate as the main component continuously withdrawn from the column bottom portion of the continuous multi-stage distillation column B1 to a continuous multi-stage distillation column B2 having a side withdrawal port, and continuously withdrawing a side-cut component ($B_s$) containing the dialkyl carbonate as the main component from the side withdrawal port,

   wherein

   in the step (I), a concentration of the dialkyl carbonate in the low-purity dialkyl carbonate mixture ($A_T$) to be fed to the continuous multi-stage distillation column B1 is from 25.00 to 95.00% by mass, and a temperature of column bottom of the continuous multi-stage distillation column B1 is 115°C or more,
   in the step (II), a concentration of the dialkyl carbonate in the column bottom component ($B_B$) to be fed to the continuous multi-stage distillation column B2 is from 99.00 to 99.95% by mass, and
   in the step (II), a purity of the dialkyl carbonate in the side-cut component ($B_s$) withdrawn from the side withdrawal port of the continuous multi-stage distillation column B2 is 99.99% by mass or more.

2. The production method according to claim 1, wherein the step (I) is performed in the presence of a compound containing Fe.

3. The production method according to claim 2, wherein a contacting surface area of the compound containing Fe with the low-purity dialkyl carbonate mixture ($A_T$) in the step (I) is $1.0 \times 10^{-3}$ $m^2 \cdot min/(kg/Hr)$ or more.

4. The production method according to claim 3, wherein the step (I) is performed in the presence of iron(II) oxide.

5. The production method according to any one of claims 1 to 4, wherein a content of a high boiling point compound in the side-cut component ($B_s$) withdrawn from the side withdrawal port of the continuous multi-stage distillation column B2 is 30 mass ppm or less.

6. The production method according to any one of claims 1 to 4, wherein a metal content in the side-cut component ($B_s$) withdrawn from the side withdrawal port of the continuous multi-stage distillation column B2 is 1 mass ppm or less.

7. The production method according to any one of claims 1 to 4, wherein a water content in the side-cut component ($B_s$) withdrawn from the side withdrawal port of the continuous multi-stage distillation column B2 is 30 mass ppm or less.

8. The production method according to any one of claims 1 to 4, wherein a total content of methanol and ethanol in the side-cut component ($B_s$) withdrawn from the side withdrawal port of the continuous multi-stage distillation column B2 is 20 mass ppm or less.

9. The production method according to any one of claims 1 to 4, wherein a content of 2-methoxy ethanol in the side-cut component ($B_s$) withdrawn from the side withdrawal port of the continuous multi-stage distillation column B2 is 50 mass ppm or less.

10. The production method according to any one of claims 1 to 4, wherein a temperature of the low-purity dialkyl carbonate mixture ($A_T$) to be fed to the continuous multi-stage distillation column B1 is set to 100 to 150°C.

11. The production method according to claim 10, wherein the low-purity dialkyl carbonate mixture ($A_T$) is heated with a heater, and a time required for feeding from the heater to the continuous multi-stage distillation column B1 is 5 minutes or less.

12. The production method according to any one of claims 1 to 4, wherein the column top component ($B_T$) continuously withdrawn from the upper portion of the column of the continuous multi-stage distillation column B1 is condensed under conditions of a reflux drum temperature of 100 to 150°C.

13. The production method according to any one of claims 1 to 4, wherein

    the continuous multi-stage distillation column B2 comprises a column upper stage straight body portion, a column lower stage straight body portion having a diameter larger than that of the column upper stage straight body portion, and a taper portion connecting the column upper stage straight body portion to the column lower stage straight body portion, and
    the side withdrawal port of the continuous multi-stage distillation column B2 is provided in the taper portion.

14. The production method according to claim 13, wherein, in the continuous multi-stage distillation column B2, a ratio of a column diameter $D_{21}$ (cm) of the column upper stage straight body portion to a column diameter $D_{22}$ (cm) of the column lower stage straight body portion satisfies the conditions of the following formula (ii) :

$$0.2 < D_{21}/D_{22} < 1.0 \ldots \text{(ii)}.$$

15. The production method according to any one of claims 1 to 4, wherein, in the continuous multi-stage distillation column B1, a residence time of liquid in column calculated by the following formula (i) is 5 minutes or longer:

Residence time of liquid in column (min) = BTM volume (volume of liquid residing in the column BTM during operation (kg))/BTM withdrawal flow rate (flow rate for withdrawal of column bottom component (kg/min))...(i).

16. The production method according to any one of claims 1 to 4, wherein, in the continuous multi-stage distillation column B2, the side-cut component ($B_s$) is withdrawn in a gaseous form.

17. The production method according to any one of claims 1 to 4, wherein an internal of the continuous multi-stage distillation column B1 is a tray and/or a packing.

**18.** The production method according to any one of claims 1 to 4, wherein a reflux ratio of the continuous multi-stage distillation column B1 is from 0.5 to 5.

**19.** The production method according to any one of claims 1 to 4, wherein a reflux ratio of the continuous multi-stage distillation column B2 is from 0.2 to 4.

**20.** The production method according to any one of claims 1 to 4, wherein, in the column bottom component ($B_B$) of the continuous multi-stage distillation column B1, a content of 2-methoxy ethanol in the dialkyl carbonate is 100 mass ppm or less.

**21.** The production method according to any one of claims 1 to 4, wherein the column bottom component ($B_B$) of the continuous multi-stage distillation column B1 is fed directly to the continuous multi-stage distillation column B2, or fed to an industry-grade dialkyl carbonate tank and then fed from the tank to the continuous multi-stage distillation column B2.

**22.** The production method according to any one of claims 1 to 4, wherein a content of a high boiling point compound in the column bottom component ($B_B$) of the continuous multi-stage distillation column B1 is 0.1 mass ppm or more.

**23.** The production method according to any one of claims 1 to 4, comprising a step of reacting a cyclic carbonate with an aliphatic monohydric alcohol to provide a low-purity dialkyl carbonate mixture ($A_T$) containing a dialkyl carbonate.

**24.** The production method according to any one of claims 1 to 4, comprising a step of continuously feeding a cyclic carbonate and an aliphatic monohydric alcohol into a continuous multi-stage distillation column A in which a catalyst is present, performing reaction and distillation simultaneously in the column, and continuously withdrawing a low-purity dialkyl carbonate mixture ($A_T$) containing a dialkyl carbonate generated and unreacted aliphatic monohydric alcohol from the upper portion of the column.

**25.** A production apparatus for a dialkyl carbonate, comprising:

a continuous multi-stage distillation column B1, to which a low-purity dialkyl carbonate mixture ($A_T$) containing an dialkyl carbonate and an aliphatic monohydric alcohol is continuously fed, from an upper portion of which a column top component ($B_T$) containing the aliphatic monohydric alcohol as the main component is continuously withdrawn, and from a lower portion of which a column bottom component ($B_B$) containing the dialkyl carbonate as the main component is continuously withdrawn, and
a continuous multi-stage distillation column B2, to which a column bottom component ($B_B$) containing the dialkyl carbonate as the main component continuously withdrawn from column bottom portion of the continuous multi-stage distillation column B1 is continuously fed, the continuous multi-stage distillation column B2 having a side withdrawal port from which a side-cut component ($B_s$) containing the dialkyl carbonate as the main component is continuously withdrawn, wherein
a temperature of column bottom of the continuous multi-stage distillation column B1 can be set to 115°C or more, and
the production apparatus is configured so as to allow a compound containing Fe to exist in the continuous multi-stage distillation column B1.

**26.** The production apparatus according to claim 25, configured so as to enable iron(II) oxide to be fed in the continuous multi-stage distillation column B1.

**27.** The production apparatus according to claim 25, wherein a material in the continuous multi-stage distillation column B1 is carbon steel.

**28.** The production apparatus according to claim 25, comprising a heater that heats the low-purity dialkyl carbonate mixture ($A_T$) to be fed to the continuous multi-stage distillation column B1.

**29.** The production apparatus according to claim 25, wherein

the continuous multi-stage distillation column B2 comprises a column upper stage straight body portion, a column lower stage straight body portion having a diameter larger than that of the column upper stage straight body portion, and a taper portion connecting the column upper stage straight body portion to the column lower

stage straight body portion, and
the side withdrawal port of the continuous multi-stage distillation column B2 is provided in the taper portion.

**30.** The production apparatus according to claim 29, wherein, in the continuous multi-stage distillation column B2, a ratio of a column diameter $D_{21}$ (cm) of the column upper stage straight body portion to a column diameter $D_{22}$ (cm) of the column lower stage straight body portion satisfies the conditions of the following formula (ii) :

$$0.2 < D_{21}/D_{22} < 1.0 \ldots (ii).$$

**31.** The production apparatus according to any one of claims 25 to 30, wherein an internal of the continuous multi-stage distillation column B1 is a tray and/or a packing.

**32.** The production apparatus according to any one of claims 25 to 30, wherein the column bottom component ($B_B$) of the continuous multi-stage distillation column B1 is fed directly to the continuous multi-stage distillation column B2, or fed to an industry-grade dialkyl carbonate tank and then fed from the tank to the continuous multi-stage distillation column B2.

[Figure 1]

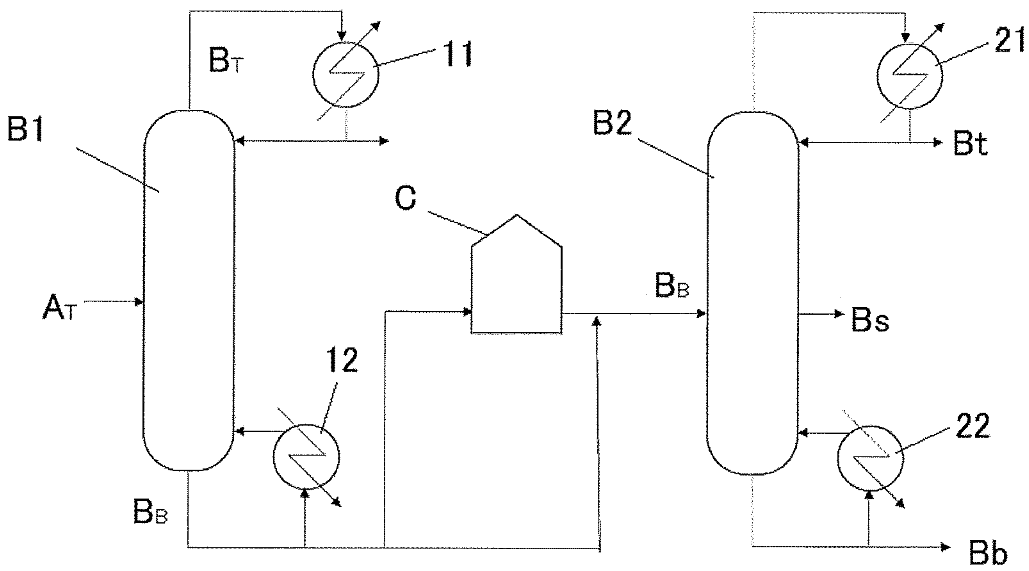


[Figure 2]

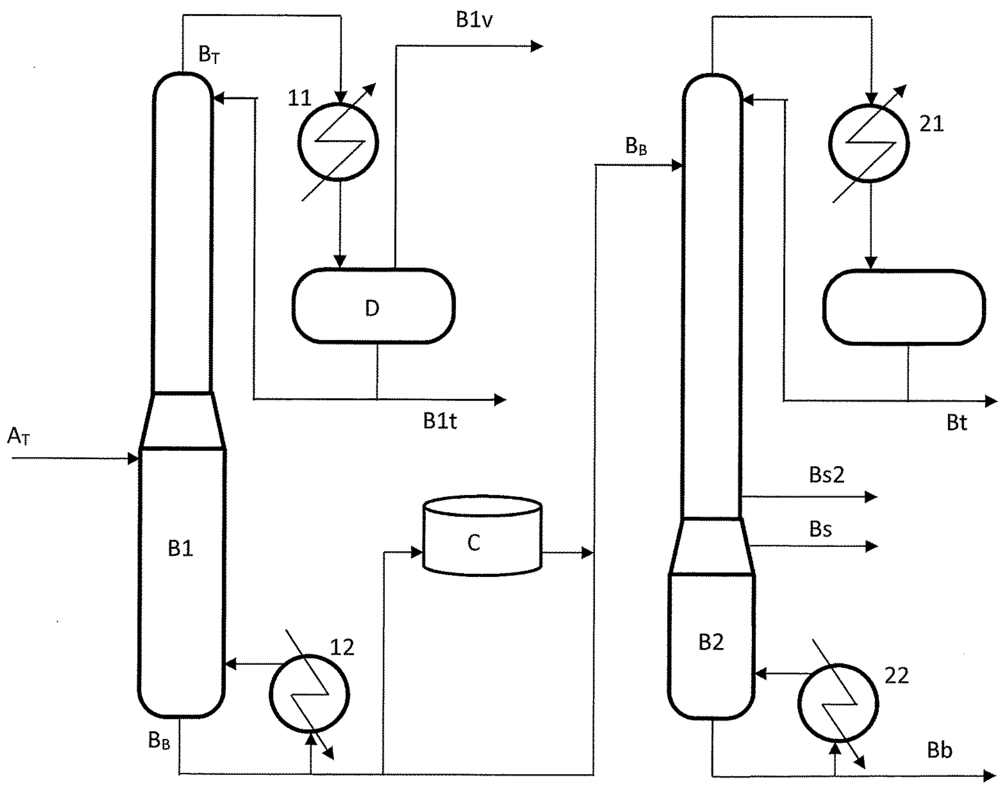

[Figure 3]

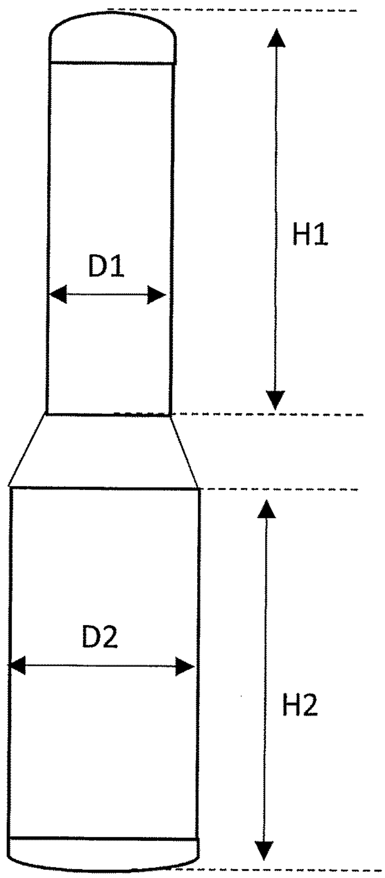

**INTERNATIONAL SEARCH REPORT**

International application No.
**PCT/JP2022/018599**

**A. CLASSIFICATION OF SUBJECT MATTER**

***C07C 68/08***(2006.01)i; ***C07C 69/96***(2006.01)i
FI: C07C68/08; C07C69/96 Z

According to International Patent Classification (IPC) or to both national classification and IPC

**B. FIELDS SEARCHED**

Minimum documentation searched (classification system followed by classification symbols)

C07C68/08; C07C69/96

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | CN 107311864 A (CHEN, Degui) 03 November 2017 (2017-11-03) claims, examples | 1-32 |
| A | CN 105384639 A (DONGYING HI-TECH SPRING CHEMICAL INDUSTRY CO., LTD.) 09 March 2016 (2016-03-09) claims, examples | 1-32 |
| A | WO 2007/074664 A1 (ASAHI KASEI CHEMICALS CORPORATION) 05 July 2007 (2007-07-05) claims, examples | 1-32 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 June 2022** | **12 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/018599**

| Patent document cited in search report | Publication date (day/month/year) | Patent family member(s) | Publication date (day/month/year) |
|---|---|---|---|
| CN 107311864 A | 03 November 2017 | (Family: none) | |
| CN 105384639 A | 09 March 2016 | (Family: none) | |
| WO 2007/074664 A1 | 05 July 2007 | US 2009/0054676 A1<br>claims, examples<br>EP 1967508 A1<br>CN 101346340 A<br>EA 200800919 A<br>BR PI0620605 A<br>KR 10-2008-0072936 A<br>TW 200734299 A<br>IN 869KO2008 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107311864
- CN 105384639